(19) **Europäisches Patentamt / European Patent Office / Office européen des brevets**

(11) **EP 4 555 918 A1**

(12) **EUROPEAN PATENT APPLICATION**
published in accordance with Art. 153(4) EPC

(43) Date of publication:
**21.05.2025   Bulletin 2025/21**

(21) Application number: **23839633.7**

(22) Date of filing: **11.07.2023**

(51) International Patent Classification (IPC):
***A61B 3/10*** (2006.01)

(52) Cooperative Patent Classification (CPC):
**A61B 3/10**

(86) International application number:
**PCT/JP2023/025587**

(87) International publication number:
**WO 2024/014461 (18.01.2024 Gazette 2024/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:  **14.07.2022   US 202263389049 P**

(71) Applicants:
• **Tokai University Educational System**
  **Tokyo, 151-0063 (JP)**
• **The Jikei University**
  **Tokyo 105-8461 (JP)**
• **Hitachi, Ltd.**
  **Tokyo 100-8280 (JP)**
• **TOPCON CORPORATION**
  **Tokyo 174-8580 (JP)**

(72) Inventors:
• **TATEMICHI Masayuki**
  **Tokyo 151-0063 (JP)**
• **FUKAI Kota**
  **Tokyo 151-0063 (JP)**
• **NAKANO Tadashi**
  **Tokyo 105-8461 (JP)**
• **TERAUCHI Ryo**
  **Tokyo 105-8461 (JP)**
• **NAKAGAWA Toru**
  **Tokyo 100-8280 (JP)**
• **AKIBA Masahiro**
  **Tokyo 174-8580 (JP)**
• **TSURI Shigetaka**
  **Tokyo 174-8580 (JP)**
• **KIKAWA Tsutomu**
  **Tokyo 174-8580 (JP)**

(74) Representative: **Gagel, Roland**
  **Patentanwalt Dr. Roland Gagel**
  **Landsberger Strasse 480a**
  **81241 München (DE)**

(54) **GLAUCOMA SCREENING METHOD, OPHTHALMIC DEVICE, PROGRAM, AND RECORDING MEDIUM**

(57)     The glaucoma screening method according to an embodiment includes the following steps. In a calculation model preparation step, a calculation model is prepared, the calculation model including a linear combination of at least one papilla parameter that represents the thickness of a retinal tissue in an optic papilla area and a linear combination of at least one macula parameter that represents the thickness of a retinal tissue in a macula area. In an OCT data generation step, the ocular fundus of an eye to be tested is subjected to OCT scanning to generate OCT data. In a measurement value calculation step, a plurality of measurement values including a papilla measurement value corresponding to each papilla parameter and a macula measurement value corresponding to each macular parameter of the calculation model are calculated on the basis of the OCT data. In a measurement value input step, the calculated plurality of measurement values are input into the calculation model. In a calculation result provision step, a calculation result output from the calculation model in accordance with the inputting the plurality of measurement values is provided.

EP 4 555 918 A1

Fig. 1

```
                    ┌─────────────┐
                    │    Start    │
                    └──────┬──────┘
                           │
   S11                     ▼
 ┌─────────────────────────────────────────────────────┐
 │ Create glaucoma risk score calculation model         │
 │ including ONH parameters and macular parameters       │
 └─────────────────────────┬───────────────────────────┘
   S12                      ▼
 ┌─────────────────────────────────────────────────────┐
 │          Generate OCT data of eye fundus              │
 └─────────────────────────┬───────────────────────────┘
   S13                      ▼
 ┌─────────────────────────────────────────────────────┐
 │ Calculate ONH measurement values and                 │
 │ macular measurement values from OCT data              │
 └─────────────────────────┬───────────────────────────┘
   S14                      ▼
 ┌─────────────────────────────────────────────────────┐
 │   Input measurement values into calculation model     │
 └─────────────────────────┬───────────────────────────┘
   S15                      ▼
 ┌─────────────────────────────────────────────────────┐
 │ Provide glaucoma risk score                          │
 │ output from calculation model                         │
 └─────────────────────────┬───────────────────────────┘
                           │
                           ▼
                    ┌─────────────┐
                    │     End     │
                    └─────────────┘
```

**Description**

[TECHNICAL FIELD]

The present disclosure generally relates to a method of glaucoma screening, an ophthalmic apparatus, a program, and a recording medium.

[BACKGROUND OF THE INVENTION]

Glaucoma is one of the main causes of blindness. The number of people with glaucoma worldwide is estimated to be more than 60 million, and is expected to continue to increase. Several epidemiological studies have revealed that many people with glaucoma have not been diagnosed and are unaware that they have glaucoma.
To prevent restriction in daily life caused by visual field loss and blindness due to glaucoma, it is important to detect the disease at an early stage and start treatment. Widely providing screening using ophthalmic examinations is considered to be an effective way to promote the early detection. However, group screening (mass screening) for glaucoma is not currently common in many countries.

[PRIOR ART DOCUMENTS]

[PATENT DOCUMENTS]

[PATENT DOCUMENT 1] Japanese Unexamined Patent Application Publication No. Hei 5-184542
[PATENT DOCUMENT 2] United States Patent Application Publication No. 2018/0025112

[BRIEF SUMMARY OF THE INVENTION]

[PROBLEM TO BE SOLVED BY THE INVENTION]

To implement mass screening for glaucoma, several issues need to be addressed. Two representative problems are described below.
The first issue is the reliability of glaucoma identification. For example, existing or conventional glaucoma identification methods that utilize image analysis have often misjudged normal eyes as having retinal thinning due to the influence of artifacts, blood vessel images, and other factors. In other words, existing or conventional methods have the problem of a high rate occurrence of false-positives. While specialists can easily distinguish false-positives caused by artifacts or other factors, mass screening is typically conducted in environments without specialists, which raises the possibility that normal eyes may be classified as suspected glaucoma cases. Such individuals would undergo further detailed examinations at a specialized hospital, but the results would naturally be negative. The occurrence of such situations not only undermines the reliability of the facility conducting the mass screening and the reliability of the screening method itself, but also degrades the quality of statistical data collected by the mass screening. In addition, for the individual being screened, it imposes unnecessary psychological, physical, financial, and time burdens. Therefore, improving the quality (precision, accuracy, etc.) of disease identification is one of the conditions for mass screening for glaucoma to be implemented.
The second issue is the difficulty and complexity of identifying normal tension glaucoma. Normal tension glaucoma is one of the types of glaucoma in which the optic nerves are damaged despite normal intraocular pressure, unlike the type caused by elevated intraocular pressure. Existing or conventional glaucoma screening methods often first rely on intraocular pressure as an indicator (biomarker) for screening, making it impossible to identify normal tension glaucoma, which does not present with elevated intraocular pressure. In order to identify normal tension glaucoma, the only method has been for a doctor to make a judgment after performing not only an intraocular pressure test but also a fundus examination and a visual field test. It is clear that this method is unsuitable for mass screening.
One objective of the present disclosure is to provide a glaucoma identification technique suitable for mass screening.

[MEANS FOR SOLVING THE PROBLEM]

One aspect example according to the present disclosure is a method of glaucoma screening comprising: preparing a calculation model that includes a linear combination of one or more optic nerve head parameters indicating retinal tissue thickness in an optic nerve head area and a linear combination of one or more macular parameters indicating retinal tissue thickness in a macular area; generating OCT data by applying an optical coherence tomography scan to a fundus of a subject's eye; calculating, based on the OCT data, a plurality of measurement values including one or more optic nerve head measurement values respectively corresponding to the one or more optic nerve head parameters and one or more macular measurement values respectively corresponding to the one or more macular parameters; inputting the plurality of measurement values into the calculation model; and providing a calculation result output from the calculation model in response to the inputting the plurality of measurement values.

Another aspect example according to the present disclosure is an ophthalmic apparatus comprising: a memory configured to retain a calculation model that is created in advance and includes a linear combination of one or more optic nerve head parameters indicating retinal tissue thickness in an optic nerve head area and a linear combination of one or more macular parameters indicating retinal tissue thickness in a macular area; an optical coherence tomography (OCT) data acquisition unit configured to acquire OCT data of a fundus of a subject's eye; a measurement value calculator configured to calculate, based on the OCT data, a plurality of measurement values including one or more optic nerve head measurement values respectively corresponding to the one or more optic nerve head parameters and one or more macular measurement values respectively corresponding to the one or more macular parameters; and a risk information generator configured to generate glaucoma risk information of the subject's eye based on the calculation model and the plurality of measurement values.

Another aspect example according to the present disclosure is a program that is capable of causing a computer including a processor and a memory to execute glaucoma screening, the program configured to cause the processor to execute: control of storing, in the memory, a calculation model that includes a linear combination of one or more optic nerve head parameters indicating retinal tissue thickness in an optic nerve head area and a linear combination of one or more macular parameters indicating retinal tissue thickness in a macular area; control of receiving optical coherence tomography (OCT) data of a fundus of a subject's eye; processing of calculating, based on the OCT data, a plurality of measurement values including one or more optic nerve head measurement values respectively corresponding to the one or more optic nerve head parameters and one or more macular measurement values respectively corresponding to the one or more macular parameters; processing of inputting the plurality of measurement values into the calculation model; and processing of providing a calculation result output from the calculation model in response to the processing of inputting the plurality of measurement values.

Another aspect example according to the present disclosure is a computer-readable non-transitory recording medium storing a program that is capable of causing a computer including a processor and a memory to execute glaucoma screening, the program configured to cause the processor to execute: control of storing, in the memory, a calculation model that includes a linear combination of one or more optic nerve head parameters indicating retinal tissue thickness in an optic nerve head area and a linear combination of one or more macular parameters indicating retinal tissue thickness in a macular area; control of receiving optical coherence tomography (OCT) data of a fundus of a subject's eye; processing of calculating, based on the OCT data, a plurality of measurement values including one or more optic nerve head measurement values respectively corresponding to the one or more optic nerve head parameters and one or more macular measurement values respectively corresponding to the one or more macular parameters; processing of inputting the plurality of measurement values into the calculation model; and processing of providing a calculation result output from the calculation model in response to the processing of inputting the plurality of measurement values.

[EFFECT OF THE INVENTION]

The exemplary embodiments according to the present disclosure is capable of providing a glaucoma identification technique suitable for mass screening.

[BRIEF DESCRIPTION OF THE SEVERAL VIEWS OF THE DRAWING]

FIG. 1 is a flowchart showing the procedure of a glaucoma screening method according to one aspect example of the embodiment.
FIG. 2 is a flowchart showing the procedure of the glaucoma screening method according to one aspect example of the embodiment.
FIG. 3 is a flowchart for describing the creation of a calculation model and the quality validation thereof in the glaucoma screening method according to one aspect example of the embodiment.
FIG. 4A is a diagram for describing the OCT measurement predictors in the glaucoma screening method according to one aspect example of the embodiment.
FIG. 4B is a diagram for describing the OCT measurement predictors in the glaucoma screening method according to one aspect example of the embodiment.

FIG. 4C is a diagram for describing the OCT measurement predictors in the glaucoma screening method according to one aspect example of the embodiment.

FIG. 5 is a table showing the characteristics of the cases and controls in the development data used for the development of the calculation model in the glaucoma screening method according to one aspect example of the embodiment.

FIG. 6 is a histogram showing the distribution of cases and the distribution of controls in relation to the OCT measurement predictors in the glaucoma screening method according to one aspect example of the embodiment.

FIG. 7 is a table showing three calculation models in the glaucoma screening method according to one aspect example of the embodiment.

FIG. 8 is a receiver operating characteristic curve of the three calculation models in the glaucoma screening method according to one aspect example of the embodiment.

FIG. 9 is a table for describing the validation results of the three calculation models in the glaucoma screening method according to one aspect example of the embodiment.

FIG. 10 is a table for describing the validation results of the three calculation models in the glaucoma screening method according to one aspect example of the embodiment.

FIG. 11 is a table for describing the validation results of the three calculation models in the glaucoma screening method according to one aspect example of the embodiment.

FIG. 12 is a block diagram illustrating the configuration of an ophthalmic apparatus according to one aspect example of the embodiment.

[DETAILED DESCRIPTION OF THE INVENTION]

Some non-limiting embodiments of the present disclosure will be described in detail with reference to the drawings.

Any known or existing techniques or technologies can be combined with any of the embodiments according to the present disclosure. For example, any matters or items freely selected from the matters or items described in any of the documents cited in the present specification can be combined with any of the embodiments of the present disclosure. In addition, any techniques or technologies known in any technical field related to the present disclosure can be combined with any of the embodiments according to the present disclosure. The matters or items combined with any of the embodiments of the present disclosure are not limited to these examples. For instance, any technical matters or items disclosed by the applicant of the present application (e.g., any technical matters or items disclosed through any means such as patent applications, academic papers, websites, or any other means) may be incorporated into the present disclosure by reference.

Two or more aspect examples according to the present disclosure may be combined at least in part.

Some of the various functions of the various elements described in the embodiments according to the present disclosure can be implemented by using a circuit configuration (circuitry) or a processing circuitry. The circuitry or the processing circuitry includes any of the followings, all of which are configured and/or programmed to execute a certain function: a general purpose processor, a dedicated processor, an integrated circuit, a central processing unit (CPU), a graphics processing unit (GPU), an application specific integrated circuit (ASIC), a programmable logic device (e.g., a simple programmable logic device (SPLD), a complex programmable logic device (CPLD), or a field programmable gate array (FPGA)), a conventional circuitry, and any combination of these. A processor is considered to be processing circuitry or circuitry that includes a transistor and/or another circuitry. In the present disclosure, circuitry, a unit, a means, or any terms similar to these is hardware configured to execute a certain function, or hardware that is programmed to execute a certain function. The hardware may be any of the various hardware described in the embodiments according to the present disclosure, or alternatively, hardware that is programmed and/or configured to execute a certain function. In the case where a processor, which may be considered as a certain type of circuitry, is employed as the hardware, then circuitry, a unit, a means, or any terms similar to these refers to an element that includes a combination of hardware and software. In this case, the software is used to configure the hardware and/or the processor.

< Overview of embodiments >

One objective of some aspect examples of the present disclosure is to propose a glaucoma identification technique suitable for mass screening. To achieve this objective, the glaucoma screening technique according to the aspect examples focuses on addressing the two aforementioned issues, namely, the reliability issue in glaucoma identification (the first issue), and the difficulty and complexity issue in identification of normal tension glaucoma (the second issue), aiming to solve or mitigate these issues. It will be understood by those skilled in the art from the embodiments described below that some aspect examples of the present disclosure solve or mitigate the first and second issues.

Embodiments according to the present disclosure are not limited to aspects addressing both of the two issues. For example, some embodiments according to the present disclosure may address only one of the two issues, may also address another issue in addition to one or both of the two issues, or may address an issue other than the two issues. In other words, embodiments of the present disclosure may aim to solve or mitigate only one of the two issues, may also aim to solve or mitigate another issue in addition to one or both of the two issues, or may aim to solve or mitigate an issue other than the two issues. Hereinafter, the terms "solving an issue" and "mitigating an issue" are used interchangeably unless otherwise specified.

Optical coherence tomography (OCT) is a non-contact, high-speed, and noninvasive imaging modality. In spite of being a relatively new technology, OCT has seen rapid adoption in the field of ophthalmology and is now used in many medical institutions, including small clinics. Therefore, OCT can be said to be an imaging modality suitable for use in mass screening.

In the embodiments of the present disclosure, OCT is used to generate a crosssectional image of the eye fundus. More specifically, the glaucoma identification in the embodiments according to the present disclosure is essentially performed solely on the basis of an image obtained using OCT (referred to as an OCT image). In contrast, various known glaucoma identification techniques essentially utilize not only an OCT image but also background information on the subject, intraocular pressure data, visual field test data, and an image obtained using an ophthalmic modality other than OCT (such as a slit lamp microscope, a fundus camera, or a scanning laser ophthalmoscope). It should be noted that even if a glaucoma identification technique exists that uses only an OCT image, this technique is considered to differ from the glaucoma identification of the embodiments according to the present disclosure at least in that it is not capable of solving the first issue and/or the second issue, and in that it does not have the configuration for solving these issues.

The type of OCT used in the embodiments of the present disclosure may be freely selected or determined. Typically, spectral domain OCT (SD-OCT) or swept source OCT (SS-OCT) may be used. In the embodiments described below, spectral domain OCT is used; however, the use of another type of OCT may yield similar configurations and effects, which will be understood by those skilled in the art.

In the embodiments according to the present disclosure, an algorithm suitable for mass screening for glaucoma using OCT is created and validated. To this end, the embodiments described below involve creating an algorithm (referred to as a calculation model) that functions to generate a glaucoma risk score from an OCT image, using key indicators selected from various indicators obtained from OCT data of the eye fundus.

Some aspects of a glaucoma screening method that can be implemented using this algorithm will now be described. These aspects are merely examples and are not intended to limit the scope of the embodiments of the present disclosure.

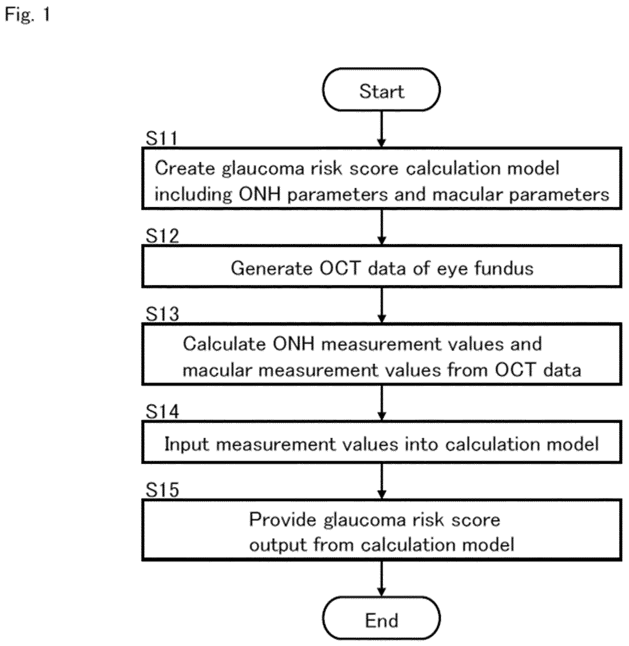

The method of glaucoma screening (method of glaucoma identification) according to one aspect is shown in FIG. 1. Note that details of each step will be described later with non-limiting examples.

The first step of the method according to the present aspect creates a calculation model of determining a glaucoma risk score from an OCT image (step S11).

The step S11 is performed by a computer that operates according to a calculation model creation program. This program may include a program that utilizes machine learning techniques or technologies. For example, development data (learning data, training data) and a mathematical model are prepared. The development data includes numerous pieces of OCT data (or many measurement values calculated from numerous pieces of OCT data) to which labels (glaucoma risk score values) are assigned. The labels are created by a specialist or another calculation model. The mathematical model is, for example, a neural network. Machine learning is then applied to the mathematical model using the development data to construct the calculation model. Some examples of how to create the calculation model will be described later.

The calculation model created in the step S11 includes a parameter related to the optic nerve head (referred to as an optic nerve head parameter) and a parameter related to the macula (referred to as a macular parameter). Therefore, the calculation model is a mathematical model configured to receive input of a measurement value corresponding to the optic nerve head parameter and a measurement value corresponding to the macular parameter, and to output a calculation result based on these measurement values.

The optic nerve head parameter is any kind of parameter measured or calculated concerning the optic nerve head, and is, for example, a parameter indicating retinal tissue thickness in an area determined for the optic nerve head (referred to as an optic nerve head area). The optic nerve head area may be, for example, any of: an area that includes at least part of the optic nerve head; an area surrounding the optic nerve head; and an area formed by combining these areas. The retinal tissue thickness represents the size or dimension (thickness) of one or more tissues constituting the retina. From a histological point of view, the retina is divided into the following 10 layers: retinal pigment epithelium, photoreceptor layer, outer limiting membrane, outer nuclear layer, outer plexiform layer, inner nuclear layer, inner plexiform layer, ganglion cell layer, nerve fiber layer, and inner limiting membrane. The retinal tissue thickness may be defined to be the thickness of one or more of these 10 layers. Optic nerve head parameters are not limited to these examples and may include any parameters that can be derived on the basis of data collected through OCT scanning applied to an area of the eye fundus determined based on the optic nerve head, such as a cup dimension (e.g., diameter, area, etc.), a disc dimension, a rim dimension, a ratio of two dimensions, optic nerve head tilt, or the like.

The macular parameters is any kind of parameter measured or calculated concerning the macula, and are, for example, a parameter indicating retinal tissue thickness in an area determined for the macula (referred to as a macular area). The macular area may be, for example, an area that includes at least part of the macula, an area surrounding the macula, or a combination of these areas. Macula parameters are not limited to these examples and may include any parameters that can be derived from data collected through OCT scanning applied to an area of the eye fundus determined based on the macula, such as a macular dimension (e.g., diameter, area, depth, etc.). The retinal tissue(s) corresponding to the macular parameter may be the same as or different from the retinal tissue(s) corresponding to the optic nerve head parameter.

The calculation model of the present aspect is expressed as a mathematical formula of a predetermined form that includes one or more optic nerve head parameters and one or more macular parameters as variables. The format of this mathematical formula may be freely selected or determined.

A calculation model of some examples includes a linear combination of one or more optic nerve head parameters and/or one or more macular parameters. In other words, the calculation model of some examples includes a linear equation (equation of the first degree) with one or more optic nerve head parameters and/or one or more macular parameters as variables.

As is well known, a linear combination of N number of parameters P1, P2, ..., PN is expressed using coefficients c1, c2, ..., cN as follows: c1P1 + c2P2 + ... + cNPN. Furthermore, a linear equation with N number of variables $x_1, x_2, ..., x_N$ is expressed as y = f(x) using coefficients $a_0$, a1, a2, ..., aN as follows: $y = a_0 + a1x1 + a2x2 + ... + aNxN$.

A mathematical formula representing a calculation models of some examples may include a term of a type other than a term of 0-th degree (constant term) and a term of the first degree (linear term). For example, a formula of a calculation model of some examples may include a polynomial of the second or higher degree. Furthermore, a formula of a calculation model of some examples may include a mathematical symbol other than the four basic arithmetic operations (namely, addition, subtraction, multiplication, and division), such as a radical symbol, a differentiation symbol, an integration symbol, or the like.

Shown below are three examples (Y (Model 1), Y (Model 2), and Y (Model 3)) of calculation models defined as multivariable linear equations whose variables include both an optic nerve head parameter(s) and a macular parameter(s). Details of each variable (e.g., TSNITlower) will be descried later.

Y (Model 1) = 14.4305 - 0.0404 × TSNITlower - 0.0303 × cpRNFLqS - 0.0304 × cpRNFLqIt - 0.0271 × cpRNFLqIn - 0.1424 × mGCIPL_IT + 1.1427 × log_mG-    [ First calculation mode ]
CIPL_STvsIT + 0.6971 × log_mGCIPL_ITvsIN + 0.0602 × mGCC_IN Y (Model 2) = 12.6694 - 0.0329 × TSNITlower - 0.0344 × cpRNFLqS - 0.0318 × cpRNFLqIt - 0.0398 × cpRNFLqIn + 1.1646 × log_mGCIPL_STxvsITx + 0.712 ×    [ Second calculation mode ]
log_mGCC_ITxvsINx

Y (Model 3) = 12.5935 - 0.066 × TSNITlower - 0.0296 × cpRNFLqIn - 0.0289 × mGCC + 0.9845 × log_mGCIPL_SNyvsINy + 0.5671 × log_mGCIPL_STyvsITy + 0.6613 ×    [ Third calculation mode ]
log_mGCC_ITyvsINy

In the present aspect, the step S11 (the creation of the glaucoma risk score calculation model) may be performed at any time point before the evaluation of the subject's eye using this calculation model (the steps S12 to S15). The created calculation model is stored in a computer that performs the evaluation of the subject's eye and/or in a storage device accessible by this computer. This storage device may be, for example, a node of a computer network, such as a local area network (LAN) to which the computer belongs, the Internet, a wide area network (WAN), or the like. Alternatively, the storage device may be a peripheral device directly or indirectly connected to the computer.

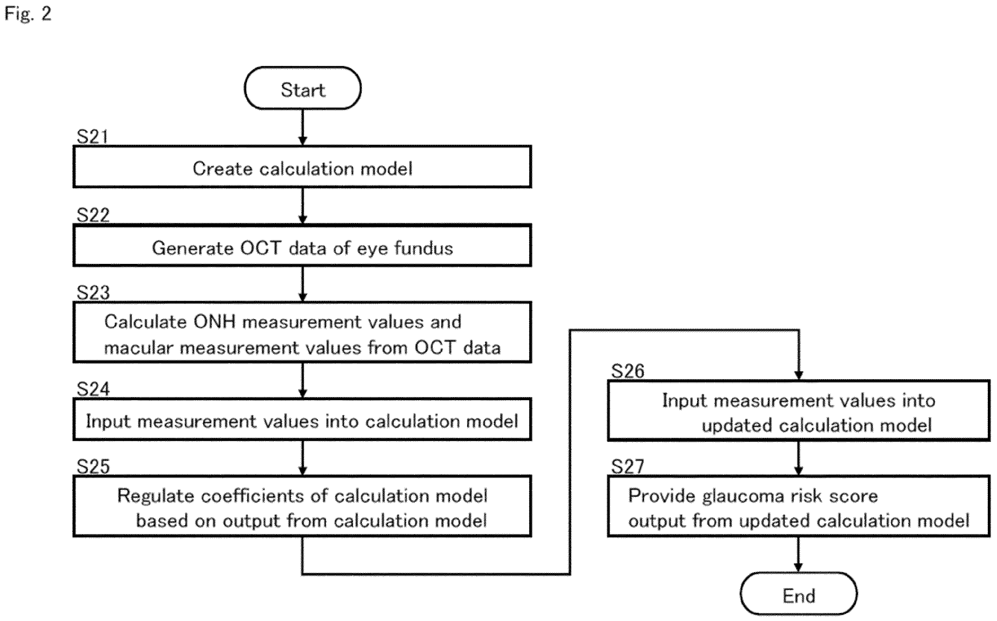

It should be noted that, as will be described later along with FIG. 2, the calculation model may be updated using data used in the evaluation of the subject's eye, which the data corresponds to the OCT data generated in the step S12 in the present aspect.

The exemplary calculation models described above are defined as mathematical formulas with only parameters derived from OCT data as their variables. However, calculation models of some other aspects may include other parameters as variables to improve the quality (e.g., accuracy, precision, reproducibility, etc.) of the calculations, for example. Examples of such additional or auxiliary parameters include the following: (1) parameters obtained from OCT data other than any of optic nerve head parameters and macular parameters; (2) parameters related to predetermined sub-tissues of the retina; (3) parameters related to tissues other than the retina (e.g. parameters related to choroid, sclera, vitreous body, corner angle, etc.); (4) parameters related to eye fundus blood vessels (e.g., vessel density, etc.); (5) parameters related to the size and/or shape of an area with a lesion or disorder; (6) parameters related to eye fundus blood flow; (7) parameters related to the subject's background information (e.g., age, sex, race, medical history, treatment history, medication history, family history, etc.); (8) parameters derived from data obtained using ophthalmic modalities other than OCT; (9) parameters related to data obtained from ophthalmic examinations (e.g., intraocular pressure data, visual field test parameters, etc.); and (10) parameters related to data obtained from tests in medical departments other than ophthalmology (e.g., blood pressure, blood test parameters, genetic test parameters, etc.).

In the step S12, OCT scanning is applied to the fundus of the subject's eye to generate OCT data. The OCT scanning is performed, for example, by an OCT apparatus (OCT scanner) of the spectral domain type.

The kind (type, format, etc.) of the OCT data generated in the step S12 is typically determined based on the processing content of the step S13. For example, the OCT data may include any of the following: data collected through the OCT scanning (collected data); image data obtained by applying imaging processing, such as Fourier transform, to the collected data (OCT image data); data obtained at an intermediate stage of the imaging processing (interim data); and data obtained by applying predetermined processing to the OCT image data (processed OCT image data).

In the case where OCT data is generated by performing processing on collected data, this processing is performed by a computer that operates according to an OCT data generation program. The OCT data generation program may be configured as a part of the program that causes the computer to execute the series of processes in the steps S12 to S15.

The number of OCT scans applied to the fundus of the subject's eye in the step S12 may be freely selected or determined number of times, once or more. In some examples, OCT scanning may be applied a plurality of times to the same region of the fundus of the subject's eye. In this case, based on a plurality of pieces of data collected from the same region, it is possible to generate an averaged image with reduced random noise (e.g., a summation average image), an OCT angiography image depicting eye fundus vessels (OCTA image), OCT blood flow measurement data showing eye fundus blood flow dynamics, or images or data of other kinds.

In some other examples, OCT scanning may be applied separately to two or more different regions of the fundus of the subject's eye to generate OCT data for the two or more regions separately. For example, OCT scanning for a region that includes an optic nerve head area (referred to as an optic nerve head area scan) and OCT scanning for a region that includes a macular area (referred to as a macular area scan) may be performed separately. In this case, OCT data for the optic nerve head area can be generated based on the optic nerve head area scan, and OCT data for the macular area can be generated based on the macular area scan.

Alternatively, a single OCT scanning may be applied to a region that includes both an optic nerve head area and a macular area (referred to as a wide area scan). In this case, both OCT data for the optic nerve head area and OCT data for the macular area can be generated based on data collected by the wide area scan.

In the next step S13, data to be input into the calculation model created in the step S11 is determined based on the OCT data generated in the step S12. As mentioned earlier, the variables of the calculation model of the present aspect include one or more optic nerve head parameters and one or more macular parameters. Therefore, in the step S13, an optic nerve head measurement value corresponding to each optic nerve head parameter and a macular measurement value corresponding to each macular parameter are calculated. Note that a measurement value other than these may be calculated as supplementary or auxiliary data.

The step S13 is performed by a computer that operates according to a measurement value calculation program. The measurement value calculation program may be configured as a part of the program that causes the computer to execute the series of processes in the steps S12 to S15.

Some aspects also use information other than the measurement values calculated from OCT data (e.g., the aforementioned supplementary or auxiliary parameters). In such aspects, a computer performs a process of acquiring that information. For example, the computer may be configured to perform a process of accessing a medical information database to obtain information about the subject. Here, examples of the medical information database include an electronic medical record system, a medical image archiving system, a medical information sharing system, and a similar database.

In the next step S14, each of the optic nerve head measurement values and each of the macular measurement values calculated in the step S13 are input into the calculation model created in the step S11. Each optic nerve head measurement value is substituted for its corresponding optic nerve head parameter (variable), and each macular measurement value is substituted for its corresponding macular parameter (variable).

In some aspect examples, in addition to the measurement values calculated in the step S13, other information is also input into the calculation model. Moreover, in some aspect examples, a result output from the calculation model based on the input of the measurement values calculated in the step S13 is adjusted (corrected) on the basis of other information.

The step S14 is performed by a computer that operates according to a measurement value input program. This measurement value input program may be configured as a part of the program that causes the computer to carry out the series of processes in the steps S12 to S15.

Based on the calculation model created in the step S11 and the measurement values (and other information) input into the calculation model in the step S14, the computer obtains a calculation result (glaucoma risk score). In the step S15, the obtained glaucoma risk score is provided.

The destination for providing the glaucoma risk score is determined in advance or decided on a case-by-case basis for each process. Examples of the destination include a user, another computer, a medical system, an ophthalmic apparatus, and a storage device. In some aspect examples, the glaucoma risk score may be displayed on a display device, transmitted to a doctor's terminal, transmitted to a subject's terminal, transmitted to a server, transmitted to a database, transmitted to an examination apparatus, transmitted to a treatment apparatus, or stored in a storage device.

The step S15 is performed by a computer that operates according to an information provision program. The information provision program may be configured as a part of the program that causes the computer to carry out the series of processes in the steps S12 to S15.

The aspect shown in FIG. 1 is configured to determine a glaucoma risk score from one or more optic nerve head measurement values and one or more macular measurement values calculated from OCT data of the fundus of the subject's eye, without requiring intraocular pressure data of the subject's eye for the evaluation of glaucoma. Therefore, the present aspect is capable of identifying normal tension glaucoma. Furthermore, as will be described in detail later, the present aspect enables highly reliable glaucoma identification. As such, the present aspect provides a glaucoma identification technique suitable for mass screening.

As mentioned earlier, some aspect examples may be configured to update the calculation model using data used in the evaluation of the subject's eye. One aspect of such a glaucoma screening method (glaucoma identification method) will be described with reference to FIG. 2.

The steps S21 (creation of the calculation model), S22 (generation of OCT data), S23 (calculation of optic nerve head measurement values and macular measurement values), and the step S24 (input of the measurement values into the calculation model) may be performed in the same manner as the steps S11, S12, S13, and S14 of FIG. 1, respectively. However, different methods or procedures may be applied.

In the next step S25, based on the calculation model created in the step S21 and the measurement values input into the calculation model in the step S24, the computer obtains a glaucoma risk score and regulates the coefficients of the calculation model based on the glaucoma risk score.

Typically, the calculation model in the present aspect is defined as a multivariable linear equation with one or more optic nerve head parameters and one or more macular parameters as variables. Each term of this multivariable linear equation is a product of a coefficient and a variable. In the present aspect, for example, the development data used in the creation of the calculation model in the step S21 is updated by adding the optic nerve head measurement value(s) and the macular measurement value(s) calculated in the step S23. The coefficients of the calculation model may be regulated by applying machine learning using the updated development data to the calculation model.

The step S25 is performed by a computer that operates according to a calculation model update program. The calculation model update program may be configured as a part of the program that causes the computer to carry out the series of processes in the steps S22 to S25 (or the steps S21 to S25). Further, the calculation model update program may be the same program as the calculation model creation program used in the step S21.

In the next step S26, each optic nerve head measurement value and each macular measurement value calculated in the step S23 are input into the calculation model updated in the step S25.

Based on the new calculation model obtained in the step S25 and the measurement values (and other information) input into the new calculation model in the step S26, the computer derives a glaucoma risk score. The step S27 performs provision of the glaucoma risk score thus obtained. The provision of the glaucoma risk score is performed in the same manner as the step S15 of FIG. 1. However, different methods or procedures may be applied.

As with the aspect shown in FIG. 1, the aspect shown in FIG. 2 can identify normal tension glaucoma and can also identify glaucoma with high reliability, making it possible to implement a glaucoma identification technique suitable for mass screening. In addition, the present aspect can update the calculation model using OCT data of the fundus of the subject's eye that is the subject of glaucoma evaluation, so that the quality of the calculation model can be improved on an ongoing basis.

Since the present aspect uses data of the subject to update the calculation model, it is considered desirable to perform processing to protect personal information of the subject. In some aspect examples, items corresponding to personal information (personal information items) are defined in advance. Examples of the personal information items include subject identifiers (subject IDs), names, addresses, public or official personal numbers, and background information. In the stage of processing specific information of a subject, the computer removes information corresponding to each personal information item from all information related to the subject, and uses only the remaining information (including OCT data and/or measurement values) to update the calculation model. Such processing increases the level of personal information protection. The processing is performed by a computer that operates according to a personal information protection program. The personal information protection program may be configured as a part of the calculation model update program mentioned above.

< Aspect examples >

The overview of the embodiments has been described above. Some aspect examples of the embodiments are described below.

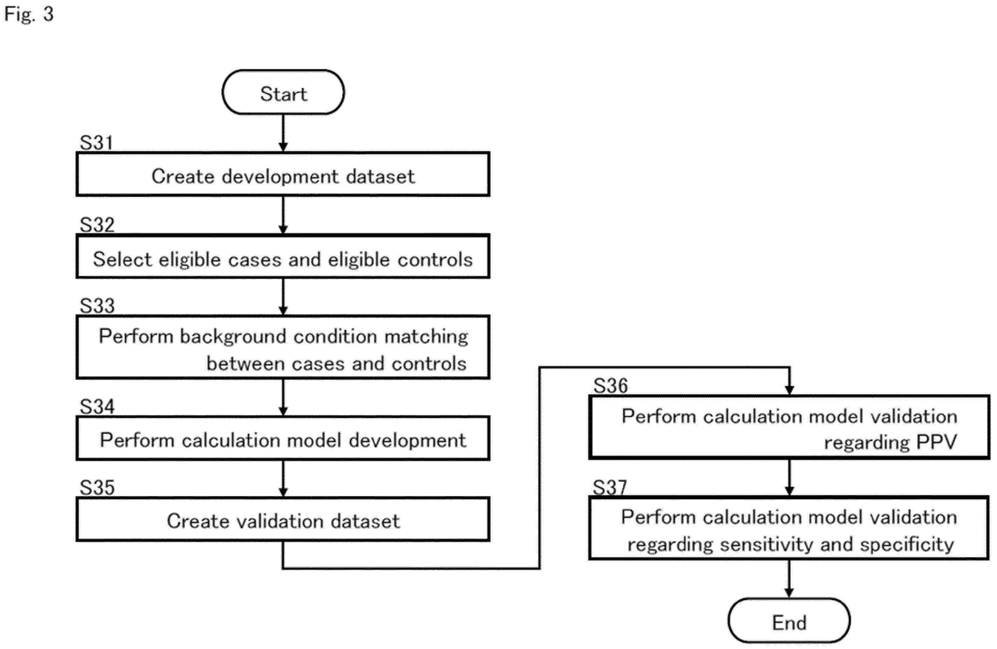

To begin with, the creation of a calculation model used in the glaucoma screening method according to one aspect example will be described along with implementation examples conducted by the inventors. The flowchart of FIG. 3 shows an overview of the procedure for creating a calculation model used in the glaucoma screening method according to the present aspect.

The first step, the step S31, is the creation of development data. The development data used in the present example includes data for 11,487 eyes of 7,572 participants. The average age of the participants is 51.3 years ($\pm$10.0 years), with an age range of 35 to 74 years. Each participant undergoes a medical history check, fundus photography using a digital fundus camera (digital retinal camera), a visual field test using a frequency doubling technology (FDT) perimeter, an automated OCT measurement using spectral-domain OCT, and an axial length measurement using an optical axial length measuring device. In other words, the data for each participant includes medical history data, a fundus photograph, visual field data, fundus OCT data, and axial length data. Note that in consideration of the detection of normal tension glaucoma, the development data in the present example does not include intraocular pressure values.

The next step, the step S32, is the selection of eligible cases and eligible controls. In the glaucoma risk score development performed in the present example, a matched case-control design is adopted. Each case is a participant who has been diagnosed with glaucoma by an ophthalmologist based on a visual field test and fundus examination. Each control is a participant who has no history of glaucoma and whose fundus photography and FDT visual field test shows no signs of glaucoma.

The next step, the step S33, is the matching of background conditions between cases and controls. In the present example, matching of sex (male or female) and matching of age (five-year age groups, five-year strata) are conducted, thereby randomly assigning one control to each case. The final development dataset used for analysis includes 284 eyes from 191 cases and 287 eyes from 277 controls.

The next step, the step S34, is the development of the calculation model using the development data obtained in the step S33. Details of the method (method or technique for statistical analysis) used to develop the calculation model in the present example, will be described later with some examples.

The next step, the step S35, is the creation of a validation dataset. The validation data is different data than the development data. In the present example, the development dataset is data obtained from participants who underwent health screenings in 2016, while the validation dataset is data obtained from participants who underwent health screenings in 2018. The validation data used in the present example includes OCT data from 9,720 eyes. Note that the validation data in the present example do not include intraocular pressure values.

The next step, the step S36, is the validation of the calculation model regarding the positive predictive value (PPV). PPV is a value defined as the probability that a result determined to be positive is truly positive. In some embodiments, PPV is defined as the probability that a "high" glaucoma risk score (described below) actually indicates the presence of glaucoma.

In the present example, the calculation model developed in the step S34 is applied to each validation data (OCT data for each of the 9,720 eyes). In addition, for validation purposes, the glaucoma risk score range is divided into three levels: "low", "medium", and "high", and then 723 eyes are randomly selected for each score level. The inventors then independently evaluated the OCT report of each of the selected eyes and classified each eye into one of four categories: "normal", "glaucoma", "ocular disease other than glaucoma", and "undetermined (requiring further detailed ophthalmic examinations)."

The validation in the present step has been conducted using the following five OCT parameters to determine glaucomatous changes: (1) average thickness of the retinal nerve fiber layer (RNFL) in a predetermined circle (a predetermined circumpapillary circular region); (2) the presence or absence of local thinning of the RNFL and the height difference between double humps in a TSNIT plot; (3) quadrant RNFL thickness chart and clock hour RNFL thickness chart; (4) retinal thickness map, ganglion cell layer ++ thickness map (GCL++ thickness map), and GCL+ thickness map for the macular area, as well as RNFL thickness map for the optic nerve head area; and (5) GCL++ thickness deviation map, GCL+ thickness deviation map, and RNFL thickness deviation map. Here, GCL+ refers to the combined layer of the ganglion cell layer and the inner plexiform layer, and is also known as the ganglion cell-inner plexiform layer (GCIPL). GCL++ refers to the combined layer of the retinal nerve fiber layer, ganglion cell layer, and inner plexiform layer, and is also known as the ganglion cell complex (GCC).

A TSNIT plot is data representing the distribution of RNFL thickness in a predetermined circumpapillary circular region. More specifically, the TSNIT plot is RNFL thickness distribution data expressed using a two-dimensional coordinate system, where the first coordinate axis represents the position (angle) in this circular region and the second coordinate axis represents RNFL thickness. The reference position (zero degrees) of the first coordinate axis in the TSNIT plot is at the temporal side (T), and the positive direction of the first coordinate axis is the direction of rotation from the temporal side (T), to the superior side (S), to the nasal side (N), and to the inferior side (I), returning to the temporal side (T) in that order.

Glaucomatous RNFL defects often show arcuate defects with a temporal boundary. For OCT reports in which the inventors did not agree on the evaluations, repeated evaluations were conducted until unanimous agreement was reached. All participants (ophthalmologists) involved in the evaluation of the validation data were blinded to the glaucoma scores and conducted evaluation in an environment where they had no access to information other than the OCT reports.

The next step, the step S37, is the validation of the calculation model regarding sensitivity and specificity. Here, "sensitivity" is a value defined as the probability of correctly determining a case as positive that should be determined as positive, while "specificity" is a value defined as the probability of correctly determining a case as negative that should be determined as negative.

For the validation of the present step, a follow-up survey was conducted on participants who were among those participated in the development data creation described in the step S31 as subjects and were judged to be suspected of having glaucoma based on fundus photography images (fundus photographs). In the follow-up survey, additional testing was performed on 129 eyes from 66 participants with a Humphrey visual field analyzer (HFA (registered trademark)) using a standard program based on the 24-2 Swedish Interactive Threshold Algorithm. The inventors evaluated the results of the Humphrey visual field tests and applied the calculation model to the dataset of the 129 eyes subject to the follow-up survey, thereby validating the consistency of the calculation model with respect to the evaluation results of the Humphrey visual field tests.

Through the procedure (steps S31 to S37) shown in the flowchart of FIG. 3 whose overview is described above, a validated calculation model of the present example is obtained.

Next, OCT measurement predictors used in the creation of the calculation model in the present example will be described. The OCT measurement predictors are predictors generated from data obtained through OCT scanning.

In the present example, the inner retinal layer thickness is obtained using OCT scanning based on a three-dimensional wide scan protocol. The three-dimensional wide scan protocol is a protocol for applying OCT scanning to a wide three-dimensional region that includes both the retinal area and the optic nerve head area. The inner retina refers to the area from the inner limiting membrane to the outer limiting membrane. From the three-dimensional dataset obtained through the OCT scanning using the three-dimensional wide scan protocol, layer thickness distribution data in the retinal area and layer thickness distribution data in the optic nerve head area are derived.

Another example may separately perform OCT scanning for a region including the retinal area and OCT scanning for a region including the optic nerve head area, generate layer thickness distribution data in the retinal area from the OCT scanning for the region including the retinal area, and generate layer thickness distribution data in the optic nerve head area from the OCT scanning for the region including the optic nerve head area. Note that the OCT scanning for the region including the retinal area may be OCT scanning for the entirety of that region, or a combination of separate OCT scanning for two or more partial regions of that region.

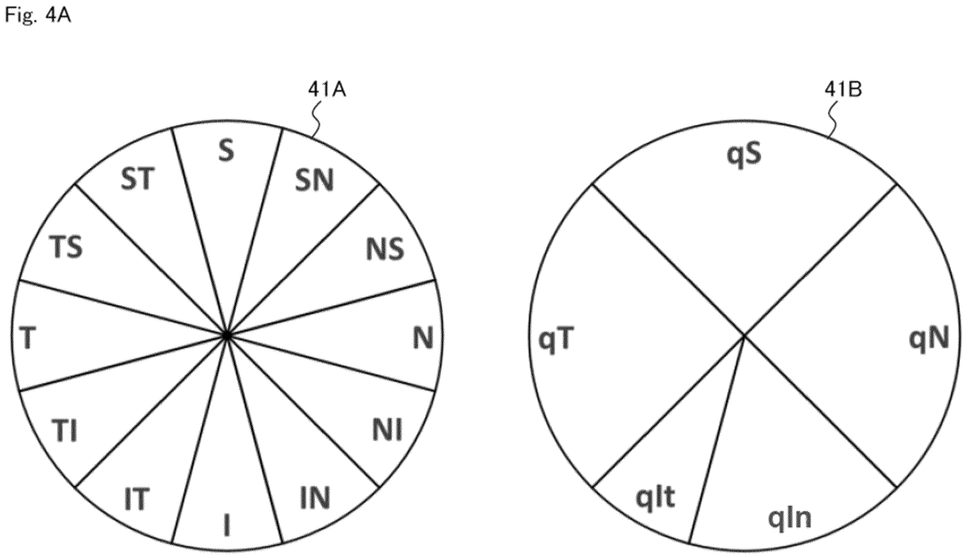

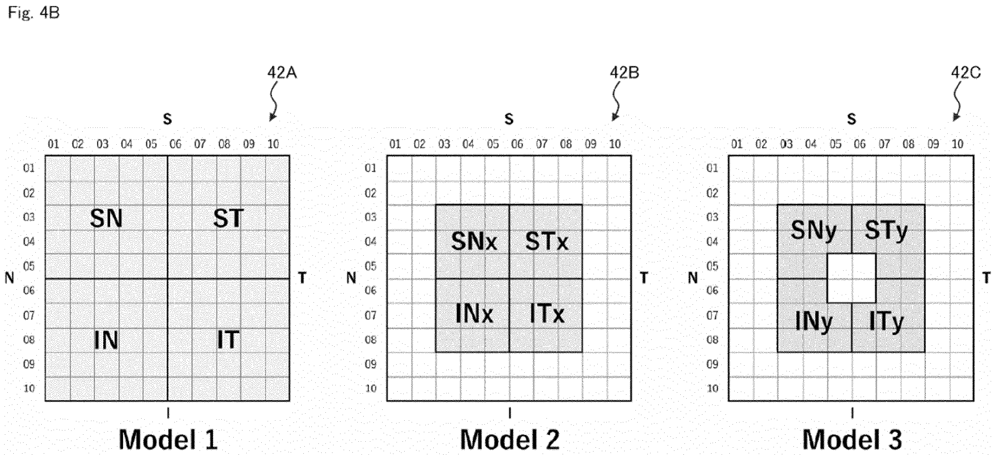

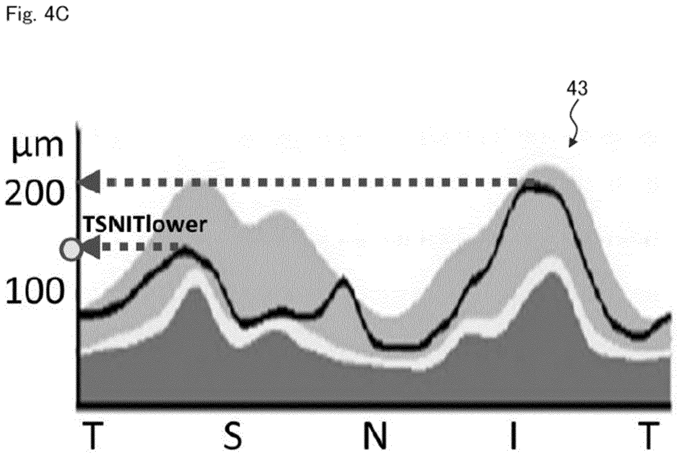

The inventors redefined the segmentation of the optic nerve head area and the grid of the macular area, and also created variables as potential predictors. The potential predictors are selected from 312 variables defined in the manner as shown in FIG. 4A, FIG. 4B, and FIG. 4C.

The first step acquires layer thickness data for the optic nerve head area (circum papillary retinal nerve fiber layer (cpRNFL) thickness data) and several types of layer thickness data for the macular area.

Of the two charts shown in FIG. 4A, the left chart 41A shows the 12 segments defined in the optic nerve head area. The optic nerve head area is a circular area (circumferential area) whose center is located at the optic nerve head center and whose radius is determined in advance. The optic nerve head area is divided into 12 segments (arc-shaped regions), each with an equal central angle of 30 degrees. The dimensions (diameter) of the optic nerve head area may be, for example, a default value or a value determined or selected according to the dimensions (diameter) of the optic nerve head.

The present example calculates the average thickness of the RNFL for each of the 12 segments of the optic nerve head area, based on the three-dimensional dataset collected by the OCT scanning conducted using the three-dimensional wide-scan protocol.

Note that as those skilled in the art would understand, the names of the 12 segments of the optic nerve head area shown in the chart 41A in FIG. 4A are defined based on the directions "superior (S)", "inferior (I)", "temporal (T)", and "nasal (N)", as well as combinations of these directions (the same applies hereafter).

Of the three charts shown in FIG. 4B, the left chart 42A shows a grid that divides the macular area into 100 segments ("10 × 10" segments). The macular area is a squareshaped area whose center is located at the fovea and whose directions are determined in advance, and is divided into 100 segments (square regions) of equal dimensions. The dimensions (sides, diagonal) of the macular area may be, for example, a default value or a value determined or selected according to the subject's eye.

The present example calculates, for each of the 100 segments of the macular area, the average thickness of the macular RNFL (mRNFL), the average thickness of the macular ganglion cell layer-inner plexiform layer (mGCIPL), and the average thickness of the macular ganglion cell complex (mGCC) based on the three-dimensional dataset acquired by means of the OCT scanning using the three-dimensional wide-scan protocol.

The second step determines variables related to the optic nerve head area. The present example defines the five regions (segments) qT, qS, qN, qIn, and qIt shown in the right chart 41B of FIG. 4A, based on the 12 segments of the optic nerve head area (cpRNFL). Furthermore, the second step creates the five variables cpRNFLqT, cpRNFLqS, cpRNFLqN, cpRNFLqIn, and cpRNFLqIt corresponding to the five regions qT, qS, qN, qIn, and qIt, respectively.

Here, cpRNFLqT represents the temporal quadrant, cpRNFLqS represents the superior quadrant, and cpRNFLqN represents the nasal quadrant. In contrast, the inferior quadrant is divided into two regions, qIn and qIt, based on the results of the analysis described below. This division of the inferior quadrant is employed based on the consideration that the inferior-temporal RNFL thickness is highly useful for detecting glaucoma.

The third step determines variables related to the macular area. In the present example, the segmentation (division into $10 \times 10$ segments) of the three types of layer thickness distributions (mRNFL thickness distribution, mGCIPL thickness distribution, and mGCC thickness distribution) mentioned above are first reconstructed in the four regions ST, SN, IT, and IN as shown in the left chart 42A of FIG. 4B, and then the variables for the macular area are defined based on these four regions. The variables in the present example are used, for example, in the first example Y (Model 1) of the calculation model mentioned earlier.

From the 100 segments of the macular area ($10 \times 10$ segments), variables are defined by excluding a peripheral part of the macular area. The present example excludes, from the 100 segments of the macular area, the 64 segments located in the area of two-segment width from the outer edge (periphery) of the macular area. Thus, attention is focused on the remaining 36 segments in the present example. Then, the present example uses the 36 segments to define variables corresponding to the four regions STx, SNx, ITx, and INx shown by the chart 42B located at the middle in FIG. 4B. The variables in the present example are used, for example, in the second example Y (Model 2) of the calculation models mentioned earlier.

Further, variables are defined by excluding both a peripheral part and a central part of the macular area from the 100 segments of the macular area. In the present example, the 64 segments located in the area of two-segment width from the outer edge of the macular area and the 4 segments located in the area of one-segment width from the center position of the macular area. Thus, attention is focused on the remaining 32 segments in the present example. Then, the present example uses the 32 segments to define variables corresponding to the four regions STy, SNy, ITy, and INy shown by the chart 42C located on the right in FIG. 4B. The variables in the present example are used, for example, in the third example Y (Model 3) of the calculation models mentioned earlier.

Each variable defined for the macular area represents the average value of the thickness (in micrometers) of the corresponding segments. For example, the RNFL thickness in segment qS (cpRNFLqS) shown in the chart 41B of FIG. 4A is defined as the average value of the RNFL thickness in the three segments S, ST, and SN in the chart 41A corresponding to the segment qS: cpRNFLqS = (cpRNFL_S + cpRNFL_ST + cpRNFL_SN) / 3 (micrometers). Similarly, the GCC thickness in the segment SN (mGCC_SN) shown in the chart 42A of FIG. 4B is defined as the average value of the GCC thickness in the 25 segments belonging to the segment SN among 100 segments divided by the grid: mGCC_SN = (mGCC 01_01 + 01_02 + ... + 05_04 + 05_05) / 25 (micrometers).

In the fourth step, variables expressing the difference in layer thickness between two regions are created. The present example creates variables expressing the difference between the superior region and the inferior region, and variables expressing the difference between the temporal region and the nasal region. These variables do not follow a normal distribution. In order to avoid an event that a calculated value becomes zero when the layer thicknesses of the two regions are equal, these variables are defined by applying the natural logarithmic transformation to the antilogarithm obtained by adding 1 to the absolute value of the difference between layer thickness values of the two regions. For example, the variable for the difference in mGCIPL thickness between the two regions ST and IT in FIG. 4B is defined as follows: log_mGCIPL_STvsIT = $\log_e$ (ImGCIPL_ST - mGCIPL_IT| + 1).

In the fifth step, which is the final step, considering the case where the difference in cpRNFL thickness between the superior region and the inferior region is large and where the thickness value on the thinner region of the two is not significant (not severe), the variable TSNITlower, corresponding to the layer thickness at the lower peak of the two peaks (called double hump) of the TSNIT plot, is defined. An example of this variable TSNITlower is shown in FIG. 4C. In typical cases, the double hump consists of a spike near the superior (S) and a spike near the inferior (I), and the variable TSNITlower is defined as the smaller one of the two layer thickness values at the peak positions of the two spikes.

Next, the statistical analysis used in the creation of the calculation model in the present example will be described.

In the present example, all variables derived from OCT measurement values are treated as continuous variables. In the statistical analysis for the present example, the optimal predictive model from the development dataset is selected by performing backward elimination using a multivariable logistic regression model with a variable reduction and augmentation method (forward-backward stepwise selection method) with the P-value of 0.1. As a result, the three calculation models Y (Model 1), Y (Model 2), and Y (Model 3) mentioned above are obtained in the present example.

The first calculation model Y (Model 1) includes all of a value related to cpRNFL, a variable related to mRNFL, a variable related to mGCIPL, and a variable related to mGCC as potential predictors. Here, each variable related to the macula is defined for the entirety of the $10 \times 10$ macular grid (covering the entire macular area) as shown in the chart 42A in FIG. 4B. For the variables related to the macula, the second calculation model Y (Model 2) uses variables defined for the macular area with the peripheral part excluded (see the chart 42B), and the third calculation model Y (Model 3) uses variables defined for the macular area with the peripheral and central parts both excluded (see the chart 42C).

Additionally, each of the three calculation models includes the average layer thickness for the entirety of the defined region (cpRNFL, mRNFL, mGCIPL, and mGCC), a variable related to the difference in layer thickness between two regions (e.g., log_mGCIPL_STvsIT), and a variable (TSNITlower) related to a spike of the TSNIT plot graph representing the layer thickness distribution around the optic nerve head (cpRNFL).

The effectiveness of the statistical analysis conducted in the present example is estimated and evaluated using the area under the receiver operating characteristic curve (AUC-ROC).

In the validation step of the present example, the intercept values and the beta values (slopes, regression coefficients) obtained from the three regression models in the development step are applied to calculate risk scores using the following two equations (Equations 1 and 2).

[Equation 1]

$$\log_e(p/(1-p)) = Y \quad <=> \quad p = \exp(Y)/(1+\exp(Y))$$

[Equation 2]

$$\text{Glaucoma screening score} = \text{round}(p \times 100)$$

Here, round(X) is the function that performs rounding of X, representing the integer closest to X.

According to the glaucoma risk score defined in this way, the scores for all participants to whom fundus OCT measurements have been applied belong to the range from 0 to 100. In other words, the range of values for the glaucoma risk score of the present example is defined to the range from 0 to 100. In addition, a higher glaucoma risk score in the present example indicates a greater likelihood of having glaucoma.

Based on the distribution of the scores, the population is classified into the following three groups: a low group (low-risk group) with scores belonging to the range from 0 to 49; a middle group (medium-risk group) with scores belonging to the range from 50 to 89; and a high group (high-risk group) with scores belonging to the range from 90 to 100. Furthermore, the positive predictive value (PPV) for screening the need for further detailed ophthalmic examinations is calculated for each group. In addition, sensitivity and specificity are evaluated using diagnostic results obtained from a subset added and reinforced with the Humphrey visual field test.

In order to clarify the influence of axial length on glaucoma screening of the present example, the improvement effect of information on axial length on the risk score is examined. Additionally, in order to confirm the accuracy in differentiation between retinal thinning due to glaucoma and retinal thinning due to high myopia, axial length is estimated based on the angle (direction) of the double hump in the circumpapillary RNFL.

To this end, first of all, the raw value (measurement value) of axial length is independently added to the third calculation model Y (Model 3), and then axial length is added to all three calculation models Y (Model 1), Y (Model 2), and Y (Model 3), followed by the selection of variables in the logistic regression as described above.

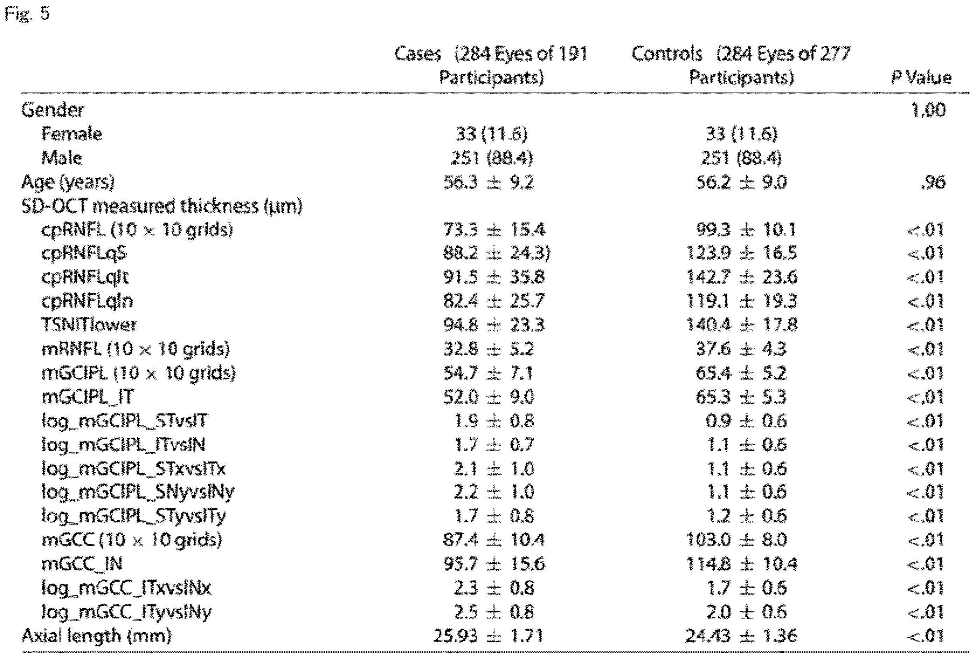

The results of this statistical analysis will now be described. FIG. 5 shows the characteristics of the cases and the controls in the development data used in the present example.

In FIG. 5, cpRNFL denotes the circumpapillary retinal nerve fiber layer, mRNFL denotes the macular retinal nerve fiber layer, mGCIPL denotes the macular ganglion cell layer-inner plexiform layer, and mGCC denotes the macular ganglion cell complex (the retinal nerve fiber layer- ganglion cell layer- inner plexiform layer in the macular area).

Furthermore, cpRNFLqIn represents the cpRNFL thickness in the inferior-nasal quadrant, cpRNFLqIt represents the cpRNFL thickness in the inferior-temporal quadrant, and cpRNFLqS represents the cpRNFL thickness in the superior quadrant.

In addition to this, log_mGCIPL_ITvsIN represents a logarithmic transformation (the first calculation model) whose antilogarithm is the difference in mGCIPL thickness between the inferior-temporal quadrant and the inferior-nasal quadrant, log_mGCIPL_SNyvsINy represents a logarithmic transformation (the third calculation model) whose antilogarithm is the difference in mGCIPL thickness between the superiornasal quadrant and the inferior-nasal quadrant, log_mGCIPL_STxvsITx represents a logarithmic transformation (the second calculation model) whose antilogarithm is the difference in mGCIPL thickness between the superior-temporal quadrant and the inferior-temporal quadrant, log_mGCIPL_STvsIT represents a logarithmic transformation (the first calculation model) whose antilogarithm is the difference in mGCIPL thickness between the superior-temporal quadrant and the inferior-temporal quadrant, and log_mGCIPL_STyvsITy represents a logarithmic transformation (the third calculation model) whose antilogarithm is the difference in mGCIPL thickness between the superior-temporal quadrant and the inferior-temporal quadrant. Further, log_mGCC_ITxvsINx represents a logarithmic transformation (the second calculation model) whose antilogarithm is the difference in mGCC thickness between the inferior-temporal quadrant and the inferior-nasal quadrant, and log_mGCC_ITyvsINy represents a logarithmic transformation (the third calculation model) whose antilogarithm is the difference in mGCC thickness between the inferior-temporal quadrant and the inferior-nasal quadrant.

In addition, mGCC_IN represents the mGCC thickness in the inferior-nasal quadrant (the first calculation model), and mGCIPL_IT represents the mGCIPL thickness in the inferior-temporal quadrant.

Also, TSNITlower represents the cpRNFL thickness at the lower spike (the lower peak) of the double hump of the TSNIT plot.

Each value shown in FIG. 5 represents a numerical value (with percentage in parentheses) or the mean $\pm$ standard deviation. The cases are participants who have been confirmed by an ophthalmologist to have glaucoma based on detailed ophthalmic examinations, while the controls are participants who have been confirmed by an ophthalmologist to have no signs of glaucoma based on both fundus photography and FDT visual field test. The P-values for ages are calculated based on the chi-square test, while the P-values for continuous variables are calculated using the t-test.

In the case group, cpRNFL values are significantly thinner than those for the normal control group, and the average values of mRNFL, mGCIPL, and mGCC over the entirety of the $10 \times 10$ grid are also significantly thinner than those for the normal control group. The Spearman's rank correlation coefficient of cpRNFL thickness between the left eye and the right eye is 0.85 (P < 0.01) in the control group and 0.24 (P < 0.01) in the case group.

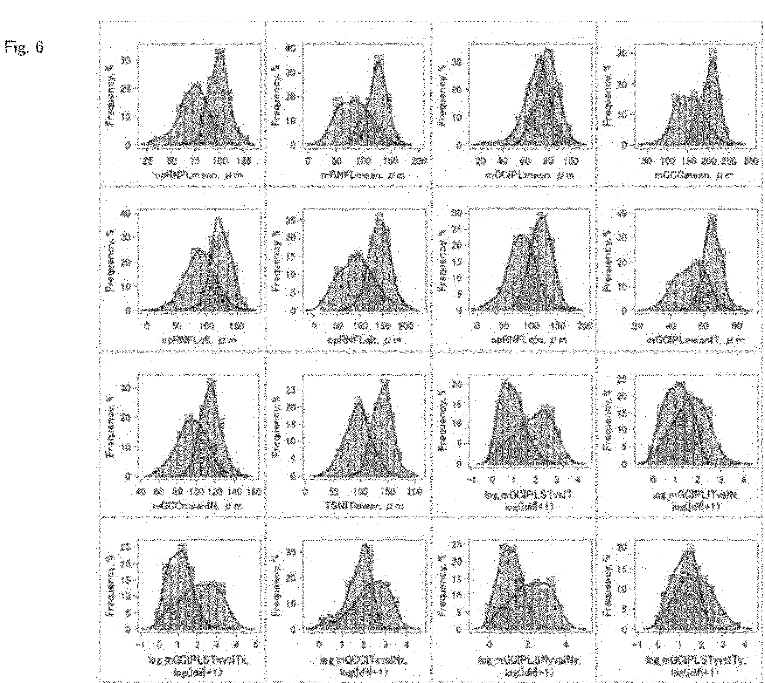

FIG. 6 shows the distribution of the cases and the distribution of the controls for each of the OCT measurement predictors in FIG. 5. Among two histograms for each OCT measurement predictors, the histogram with the higher peak corresponds to the distribution for the normal control group, while the histogram with the lower peak corresponds to the distribution for the case group.

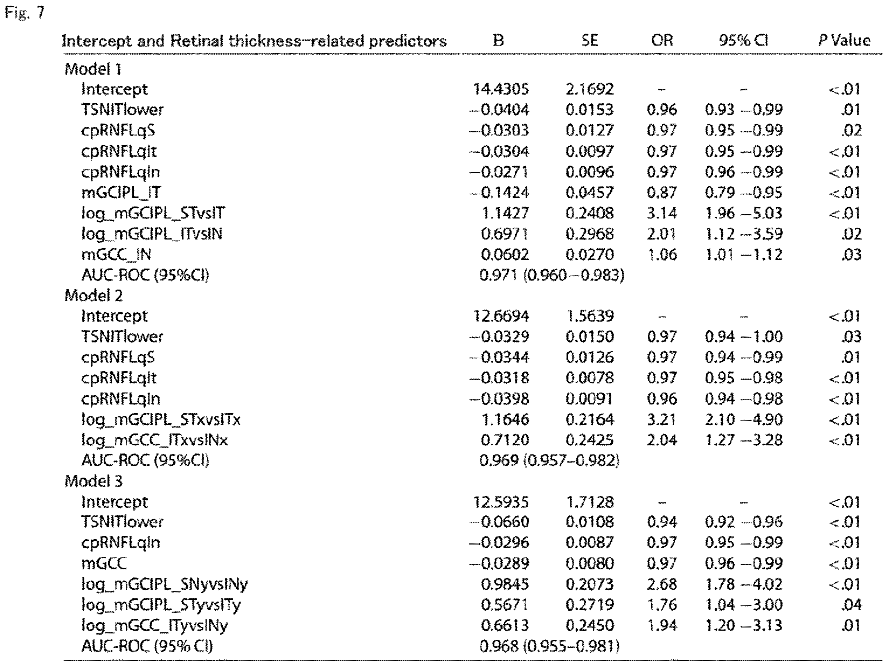

FIG. 7 shows the diagnostic model for glaucoma screening developed in the present example. FIG. 7 presents information on the intercepts and information on the retinal thickness-related predictors for each of the three calculation models Y (Model 1), Y (Model 2), and Y (Model 3). In FIG. 7, "B" denotes the slope (beta, regression coefficient), "SE" denotes the standard error, "OR" denotes the odds ratio, "CI" denotes the confidence interval, and "AUC-ROC" denotes the area under the receiver operating characteristic curve.

After selecting the variables in the logistic regression model, negative regression coefficients are generally obtained for the predictors of layer thickness in each region (cpRNFLqS, cpRNFLqIt, cpRNFLqIn, mGCIPL_IT, mGCC, and TSNITlower). This result indicates that the thicker retinal layer in a selected region is associated with a lower likelihood of having glaucoma.

In contrast, positive regression coefficients are obtained for all predictors related to the difference between the superior region and the inferior region (log_mGCIPL_STvsIT, log_mGCIPL_STxvsITx, log_mGCIPL_SNyvsINy, log_mGCIPL_STyvsITy), and for all predictors related to the difference in retinal layer thickness between the temporal region and the nasal region (log_mGCIPL_ITvsIN, log_mGCC_ITxvsINx, log_mGCC_ITyvsINy). This result indicates that larger differences in retinal layer thickness between the superior and inferior regions and/or between the temporal and nasal regions are associated with a higher likelihood of having glaucoma.

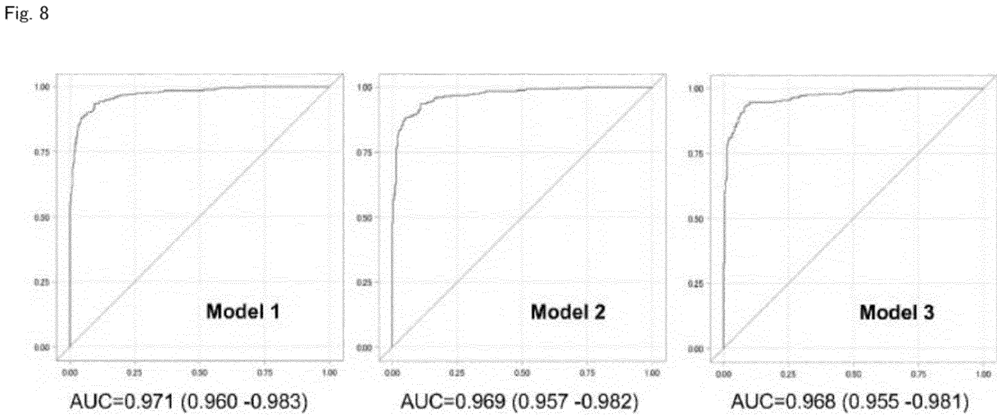

Additionally, the predicted AUC-ROC values for all three calculation models Y (Model 1), Y (Model 2), and Y (Model 3) are approximately 0.97 (see also the three ROC curves shown in FIG. 8). This result indicates that there is no significant difference between the three calculation models.

The inventors applied the glaucoma risk scores constructed in this way to the validation dataset (9,720 eyes of 6,006 participants). The glaucoma risk scores are calculated using the above-mentioned [Equation 1] and [Equation 2]. Specifically, the glaucoma risk scores can be calculated using the three calculation models Y (Model 1), Y (Model 2), and Y (Model 3).

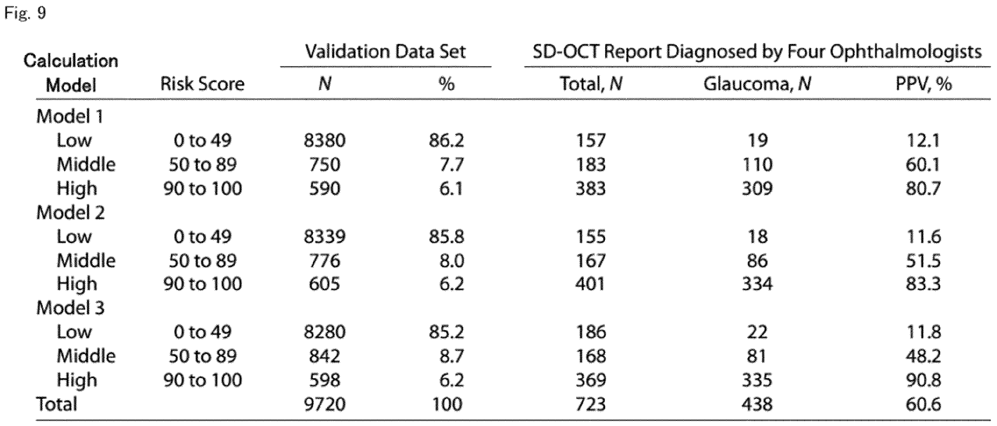

The results of the validation for the positive predictive value of the three calculation models will now be described with reference to FIG. 9. FIG. 9 shows the distribution of glaucoma risk scores obtained using the three calculation models and the positive predictive values (PPVs) regarding the need for further detailed ophthalmic examinations.

FIG. 9 shows the results of the classification of the 9,720 risk scores obtained by applying each calculation model (Model 1, Model 2, Model 3) to the 9,720 eyes in the validation dataset into the three levels of "Low," "Middle," and "High" described earlier. According to the results, the high-risk group (risk score of 90 or higher) accounted for 6.1% of the population for the first calculation model Y (Model 1), 6.2% for the second calculation model Y (Model 2), and 6.2% for the third calculation model Y (Model 3).

In order to conduct the validation of the positive predictive value (and negative predictive value), two-thirds of the high-risk group, one-fifth of the medium-risk group, and 2% of the low-risk group were randomly selected from all participants, and then four ophthalmologists conducted assessment of each selected participant based on the OCT report of that participant. As a result, the positive predictive value for screening of the high-risk group was 80.7%, 83.3%, and 90.8% for the first, second, and third calculation models, respectively. In addition, the negative predictive value in the low-risk group was 87.9%, 88.4%, and 88.2%, respectively. These results indicate that the third calculation model Y (Model 3) has the highest accuracy for glaucoma screening among the three calculation models.

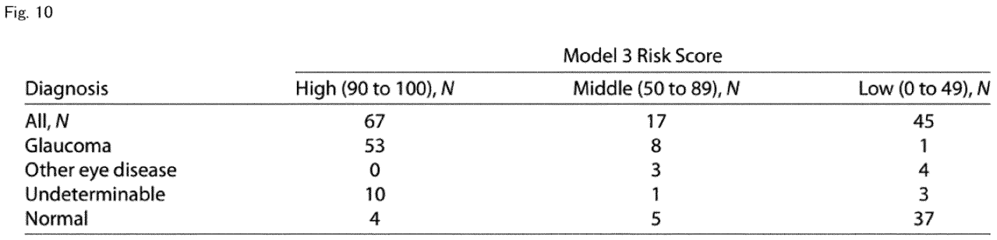

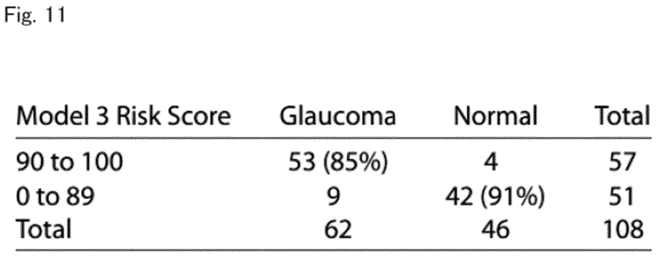

Next, the results of the validation of the third calculation model Y (Model 3) in terms of sensitivity and specificity are described with reference to FIG. 10 and FIG.11. FIG. 10 shows the results of SD-OCT examination and the Humphrey visual field test performed on participants (129 eyes), among those participated in the development data creation, who were judged to be suspected of having glaucoma based on the interpretation of fundus photographs. FIG. 10 also shows a comparison of the results of these examinations with the glaucoma risk scores from the third calculation model. Further, FIG. 11 shows the sensitivity and specificity of the risk scores in glaucoma screening using the third calculation model.

As shown in FIG. 10, the high-risk score group (90 or higher) consisted of 67 eyes according to the third calculation model. Of the 67 eyes, 53 eyes were diagnosed with glaucoma, 10 eyes were deemed undeterminable based on OCT and the Humphrey visual field test, and 4 eyes were determined to be normal.

Additionally, as shown in FIG. 11, when participants with diseases other than glaucoma were excluded, the sensitivity of the risk scores from the third calculation model was 85% (85.4%), and the specificity was 91% (91.3%).

In the false-positive group, two cases were suggestive of preperimetric glaucoma (PPG), and the optic nerve could not be tracked during the OCT scanning for two cases. In the false-negative group, two cases presented with mild retinal nerve fiber layer defects (NFLD), two cases presented with narrow NFLDs, and three cases exhibited diffuse thinning of the retinal layer due to myopia. Additionally, two cases were diagnosed with glaucoma; the scores of the two cases were 89 and 88, respectively, both of which were very close to the threshold of 90 between high risk and medium risk scores.

The Spearman's rank correlation coefficients between the risk scores from the third calculation model, and the mean deviation and the pattern standard deviation from the Humphrey visual field test, were -0.503 ($P < 0.001$) and 0.618 ($P < 0.001$), respectively. In the cases with high risk scores and confirmed glaucoma, the mean deviation and the pattern standard deviation (mean $\pm$ standard deviation) were $-6.80 \pm 5.91$ and $6.42 \pm 4.64$, respectively. In the nine cases with low or medium risk scores (< 90) and confirmed glaucoma (that is, false-negative cases), the mean deviation and the pattern standard deviation (mean $\pm$ standard deviation) were $-1.96 \pm 1.53$ and $3.02 \pm 0.76$, respectively. A significant difference between these two groups was confirmed by the t-test.

The inventors also examined the improvement effect of axial length on the risk score. First, the raw value (measurement value) of axial length was independently added to the third calculation model. As a result, the odds ratio (95% confidence interval) of axial length was 1.30 (95% confidence interval: 1.02 to 1.65). However, no improvement in accuracy was observed.

Furthermore, values of axial length were added to the variables related to OCT, and variable selection for logistic regression was performed for the first, second, and third calculation models. As a result, the odds ratios for axial length were 1.52 (95% confidence interval: 1.21 to 1.91), 1.56 (95% confidence interval: 1.32 to 2.10), and 1.52 (95% confidence interval: 1.20 to 1.93) for the first, second, and third calculation models, respectively. In this case, no improvement in accuracy was observed.

In addition, in the variable selection for all three calculation models, the estimated axial length angle of the double hump presented by the circumpapillary RNFL was not selected as a significant variable.

The glaucoma risk score of the present aspect described above was obtained using SD-OCT and can be used for population-based mass screening for glaucoma.

The development of the glaucoma risk score in the present aspect refers to the diagnostic logic of glaucoma specialists. This diagnostic logic includes the following seven items: (1) the absolute value of the thickness of each layer in the entire region, or the absolute value of the thickness of each layer in separate regions; (2) the difference in the thickness of the circumpapillary RNFL in the vertical (superior-inferior) direction; (3) the difference in the thickness of each layer in the macular area in the vertical direction and the difference in the thickness of each layer in the macular area in the horizontal (temporal-nasal) direction; (4) wedge-shaped localized optic nerve fiber layer defect (NFLD) around the optic nerve head; (5) the difference in the double hump pattern of the circumpapillary RNFL thickness distribution (TSNIT plot); (6) the layer thickness value at the lower peak in the double hump pattern of the circumpapillary RNFL thickness distribution (TSNIT plot); and (7) the value of axial length estimated from the angle (direction) of the double hump pattern of the circumpapillary RNFL.

In the present aspect, the following three patterns were considered for the macular area: (A) the first pattern using data from the entire macular area (the chart 42A of FIG. 4B, the first calculation model); (B) the second pattern using data from the region with the peripheral part of the macular area excluded in order to avoid the influence of the relatively large blood vessels in the eye fundus (the chart 42B, the second calculation model); and (C) the third pattern using data from the donut-shaped region formed by excluding the peripheral part of the macular area as in the second pattern, and also excluding the central part of the macular area where abnormalities do not occur (or rarely occur) in early glaucoma (the chart 42C, the third calculation model).

In all of the algorithms of the three calculation models according to the present aspect, the odds ratio of variables related to the vertical and horizontal differences in each layer is the most influential indicator of glaucoma. Among these three calculation models, the third calculation model provides the highest sensitivity and specificity in risk assessment using only SD-OCT data.

The validation step of the present aspect includes two steps in order to consider the following two objectives. The first objective is to develop a score that enables automatic detection of glaucoma findings using OCT, and the second objective is to provide glaucoma screening. In order to achieve these objectives, OCT data was interpreted by glaucoma specialists in the first validation, and glaucoma diagnosis was evaluated using a Humphrey visual field analyzer in the second validation. Then, cases with evenly divided risk scores were selected, and cases that were difficult to diagnose as having glaucoma were added, taking into consideration the importance of accurate judgment of slight changes in early stage glaucoma for mass screening. The validation process of the present aspect designed in this way is based on the assumption of the situation in actual mass examination (population-based mass screening) in which an ophthalmologist makes judgments on the need for additional detailed glaucoma examination in hospital.

In addition, in the present aspect, a panel of experts blinded to the risk scores was formed for the purpose of glaucoma screening. In practice, necessary decisions were made by unanimous agreement among the four ophthalmologists as mentioned above.

In order to conduct validation of the accuracy of the risk score using real-world data, cases with data of low image quality were intentionally included in the dataset used in the validation of the present aspect. The causes of the low image quality include poor fixation, blinking, eye movement, and small pupils. In some of the cases, it was difficult to interpret the glaucoma findings due to the influence of artifacts, segmentation errors, or optic nerve tracking errors. However, the data was reviewed by the four ophthalmologists until a consensus was reached to make a final decision. The risk score of the present aspect obtained in this manner is well related to the judgment required for detailed examination of glaucoma.

Further, the validity (effectiveness) of the risk scores was examined in the present aspect using ophthalmological diagnoses based on the Humphrey visual field test and OCT reports. As a result, the risk scores in the present aspect demonstrated a sensitivity of 85.4% and a specificity of 91.3%, confirming their high performance and accuracy.

In the present aspect, four cases with high risk scores and with no visual field abnormalities were regarded as false-positive cases. Some of the false-positive cases were judged likely to be preperimetric glaucoma with retinal nerve fiber layer defects detected by OCT. Although a main aim in the present aspect is to detect early and moderate stages of glaucoma, it is believed that the present aspect can also be useful for identifying preperimetric glaucoma.

Nine glaucoma cases showed low risk scores that were false-negatives cases. Consideration of these false-negative cases revealed that the risk score tended to be low for cases with a mild RNFL thinning, the width of the retinal nerve fiber layer defect is narrow, or the retinal layer shows diffuse thinning owing to myopia.

In recent years, researches on algorithm-based artificial intelligence and visualization of deep learning models have been actively pursued. However, glaucoma identification techniques suitable for mass glaucoma screening have not yet been put into practical use due to issues such as the black-box nature of algorithms and the complexity of implementation in mass screening. As described above, the algorithm according to the present aspect is capable of detecting glaucoma with high reliability and also capable of detecting normal tension glaucoma. In addition, by installing simple calculation software in existing OCT apparatuses and computers, it is possible to implement the provision of a glaucoma risk score. These facts make the algorithm according to the present aspect suitable for practical use in mass screening for glaucoma.

Furthermore, a cutoff value can be adjusted or determined in the present aspect depending on conditions such as the prevalence of glaucoma in the target population for mass screening. Here, the cutoff value refers to a threshold value for dividing the range of risk scores on the basis of the degree of risk. In the above example, the lower limit (cutoff value) of the risk score for determining the need for further detailed examination is set to 90. The risk score in this example has been shown to provide screening with a high positive predictive value, and in practice, 6% of the approximately 9,000 data sets fell into the target group for glaucoma which had a prevalence rate of 5%.

Two glaucoma cases in the validation dataset used for sensitivity and specificity validation were judged to be false negatives; however, considering that their risk scores were 88 and 89 points, it is possible to improve the sensitivity by adjusting (slightly lowering) the cutoff value. Since a high positive predictive value is considered the most crucial feature in mass screening, the calculation model according to the present aspect can be said to have both high feasibility and high reliability.

As described above, the present aspect provides an OCT screening algorithm suitable for glaucoma screening purposes. Given the irreversible and progressive characteristics of glaucoma, it is extremely important to observe changes over time. Therefore, it is considered more appropriate to express the findings of the present aspect in terms of a risk score rather than in terms of setting a clear cutoff value.

In eyes with severe myopia, the axial length may elongate, leading to deformation of the eyeball. OCT images of such eyes may cause degradation in the reliability of data used for glaucoma screening. As the degree of myopia increases, it becomes more difficult to distinguish between myopia and glaucoma. On the other hand, the distribution of RNFL thickness significantly changes with axial length, regardless of glaucoma. As previously mentioned, even when using the estimated axial length derived from the angle (direction) of the double hump in the TSNIT plot, there was no improvement in the accuracy of the risk score. Similarly, taking the raw data for axial length into consideration did not enhance the quality of the risk score.

The present aspect has strengths. Some examples of the strengths are described below.

The first strength is that a large-scale dataset, which is larger in scale than before, was used to construct the calculation model, with ophthalmologists actually interpreting numerous images as the basis for analysis. In other words, the construction of the glaucoma risk score algorithm according to the present aspect was carried out by using a dataset that is highly suitable in both quality and quantity (a high-quality and large-scale dataset).

The second strength is that all the data used to construct the algorithm according to the present aspect is population-based, free from hospital bias (e.g., variations in the average severity of disease based on the size, history, location, etc. of hospitals).

The third strength is that the algorithm according to the present aspect does not simply provide glaucoma diagnostic results (risk scores) as outputs from a black box, but can explain the reasons for those results based on ophthalmological perspectives. This enables, for example, an improvement in the quality of informed consent.

The fourth strength is that the algorithm according to the present aspect was constructed using data from participants who received appropriate follow-up care at the medical institutions where the inventors work. For example, the inventors considered the progression of glaucoma and conducted the final validation of the algorithm according to the present aspect based on the results of tests performed at a timing after an appropriate period following the initial consultation (in practice, four years after the initial visit).

The main purpose of the glaucoma risk score according to the present aspect is not to provide a definitive diagnosis of glaucoma, but rather to offer information (decision-support information) that helps doctors decide whether or not a detailed examination at a hospital is necessary for the subject. As described above, the glaucoma risk score according to the present aspect can provide a high-quality glaucoma identification technique suitable for mass screening.

It should be noted that the intended uses of the glaucoma risk score according to the present aspect are not limited to the provision of the decision-support information mentioned above (typically for mass glaucoma screening). For example, the glaucoma risk score according to the present aspect can also be used for final diagnosis or treatment of glaucoma.

< Ophthalmic apparatus >

The following provides a description of some ophthalmic apparatuses that can serve as non-limiting aspect examples of the glaucoma screening method according to the embodiments described above. An ophthalmic apparatus according to any of the aspect examples may be combined, at least in part, with any matters or items related to the glaucoma screening method according to any of the embodiments. Also, any known or existing techniques or technologies may be combined with an ophthalmic apparatus according to any of the aspect examples.

The ophthalmic apparatus according to the present aspect is used, for example, in mass screenings for ophthalmic diseases including glaucoma, and generates information indicating glaucoma risk (referred to as glaucoma risk information) of the subject's eye by processing eye fundus OCT data using a calculation model.

The calculation model in the present aspect may be any of the previously described first calculation model Y (Model 1), second calculation model Y (Model 2), or third calculation model Y (Model 3), but is not limited to these.

In some examples, two or more calculation models may be selectively used. The method of selecting the calculation model may be freely selected or determined. For example, a system may be built having the following two functions in order to ensure consistency in the screenings: the function of recording the type of calculation model applied to each target (each subject's eye, each subject, each group, etc.) for each screening; and the function of notifying, before performing a new screening, the facility conducting the new screening (or the ophthalmic apparatuses used to conduct the new screening) of the type of calculation model used in past screenings. In addition, a system can be designed to have the function of notifying the facility of the introduction of a new calculation model and/or the version upgrade of an existing calculation model at the time of the implementation, thereby improving the quality of screening.

The method used by the ophthalmic apparatus in the present aspect to acquire fundus OCT data may be freely selected or determined. The ophthalmic apparatus of some examples may include an OCT scanner that applies OCT scanning to the fundus of the subject's eye. In addition, the ophthalmic apparatus of some examples may include an element that acquires, from another device or apparatus, OCT data generated by applying OCT scanning to the fundus of the subject's eye using another ophthalmic apparatus (ophthalmic OCT apparatus) including an OCT scanner. Here, the another device or apparatus may be, for example, the ophthalmic OCT apparatus that has generated the OCT data, a medical image archiving system (e.g., a Picture Archiving and Communication System (PACS)) that retains the OCT data, or a recording medium on which the OCT data is recorded. The element that acquires the OCT data from the another device or apparatus may be, for example, a communication device for performing data communication with the ophthalmic OCT apparatus or the medical image archiving system, or a drive device that reads out the OCT data from the recording medium.

The glaucoma risk information in the present aspect may be information that expresses any type of glaucoma risk in any form or manner. The glaucoma risk information in some examples may include the glaucoma risk score described above, and/or it may include information indicating the degree of glaucoma risk (e.g., high risk, medium risk, low risk, etc.).

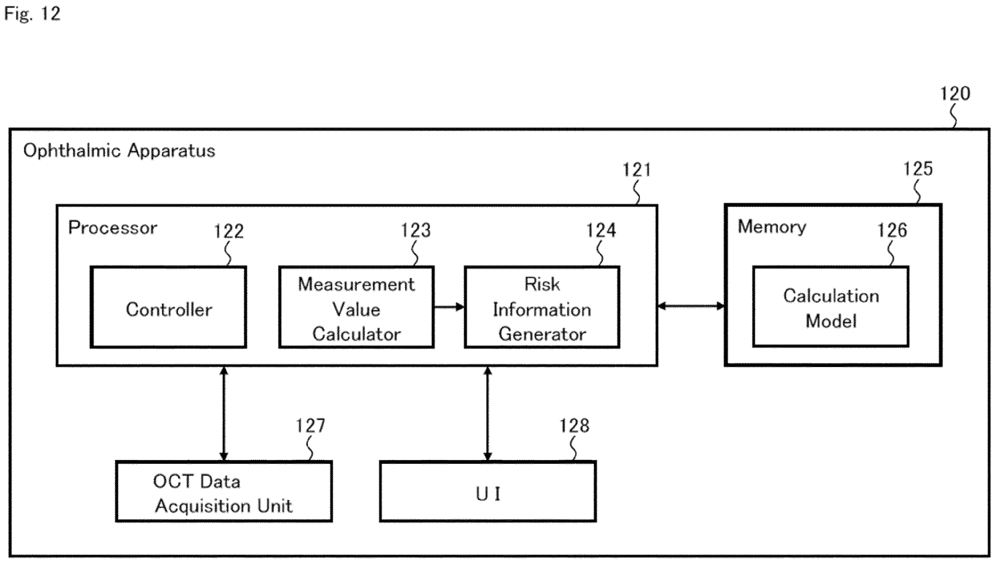

FIG. 12 shows an example of the configuration of the ophthalmic apparatus according to the present aspect. The ophthalmic apparatus 120 includes the processor 121, the memory 125, the OCT data acquisition unit 127, and the user interface (UI) 128.

The processor 121 is configured to perform various kinds of processes based on a program installed in the ophthalmic apparatus 120 and/or a program stored in a device accessible by the ophthalmic apparatus 120. The processor 121 can perform, for example, control of each element of the ophthalmic apparatus 120, control of peripheral devices of the ophthalmic apparatus 120, and processing of various kinds of data.

The memory 125 serves as a storage part of the ophthalmic apparatus 120. The memory 125 includes a storage device of any form, and may include, for example, a main storage device (primary storage device such as a main memory, cache memory, or working memory), an auxiliary storage device (secondary storage device such as a hard disk drive, flash memory, or RAM disk), a tertiary storage device, offline storage, and a register.

The memory 125 retains various kinds of data processed by the ophthalmic apparatus 120. The memory 125 stores the calculation model 126. The calculation model 126 may be any of the calculation models according to the embodiments described above. The calculation model 126 may be a linear equation that includes a linear combination of one or more optic nerve head parameters indicating retinal tissue thickness in the optic nerve head area and a linear combination of one or more macular parameters indicating retinal tissue thickness in the macular area.

The calculation model 126 is created in advance and stored in the memory 125. For example, the calculation model 126 may be a calculation model of any form created by, at least in part, employing the glaucoma screening method according to the above-mentioned embodiments. The calculation model 126 may include at least one of the first calculation model Y (Model 1), the second calculation model Y (Model 2), and the third calculation model Y (Model 3).

The OCT data acquisition unit 127 acquires OCT data of the fundus of a living eye (subject's eye) that is a target of glaucoma screening using the ophthalmic apparatus 120. The OCT data acquisition unit 127 may include, for example, the aforementioned OCT scanner and/or the aforementioned element that acquires OCT data from another device or apparatus.

Similar to existing or conventional OCT scanners, the OCT scanner includes, for example, a light source, a beam splitter, a sample arm, a reference arm, a beam combiner, and a photodetector. Here, the beam splitter splits light emitted from the light source into measurement light and reference light. The sample arm projects the measurement light onto the fundus of the subject's eye and directs the return light (reflected light, backscattered light, etc.) of the measurement light from the subject's eye. The reference arm guides the reference light. The beam combiner superposes the return light of the measurement light with the reference light to generate interference light. The photodetector detects the interference light and generates an electrical signal (also referred to as detection signal). The sample arm is provided with an optical deflector (also referred to as optical scanner) such as a galvanometer scanner to project the measurement light onto a plurality of different positions on the fundus of the subject's eye.

Furthermore, the OCT scanner includes an image data construction unit (image data construction circuit, image data construction circuitry) that applies signal processing to the detection signal generated by the photodetector to construct OCT data (typically OCT image data). The image data construction unit, for example, generates a reflection intensity profile (A-line profile) for each A-line from a spectral distribution based on data for each projection position (scan point, A-line) of the measurement light, as in existing or conventional methods. The image data construction unit then visualizes each A-line profile to generate a plurality of pieces of A-scan image data and arranges the plurality of pieces of A-scan image data in accordance with the scan pattern (arrangement of the plurality of scan points). The signal processing for generating the A-line profile includes noise reduction (denoising), filtering, and fast Fourier transform (FFT).

The OCT modality used in the OCT scanner may be freely selected or determined, and for example, spectral domain OCT or swept-source OCT may be used. Spectral domain OCT is an OCT modality that performs the following processes: a process of splitting light emitted by a low coherence light source (broad band light source, wide band light source) into measurement light and reference light; a process of projecting the measurement light onto an object; a process of generating interference light by superposing return light of the measurement light from the object and the reference light; a process of detecting a spectral distribution of the generated interference light with a spectrometer; and a process of constructing image data by applying data processing including Fourier transform to the spectral distribution detected.

On the other hand, swept source OCT is a technique including the following processes: a process of splitting light emitted by a wavelength tunable light source into measurement light and reference light; a process of projecting the measurement light onto an object; a process of generating interference light by superposing return light of the measurement light from the object and the reference light; a process of detecting the generated interference light with a photodetector; and a process of constructing image data by applying data processing including Fourier transform to detection data collected corresponding to wavelength sweeping and scanning with the measurement light.

In short, spectral domain OCT can be said to be an OCT modality of acquiring a spectral distribution of interference light in a space-divisional manner while swept source OCT can be said to be an OCT technique of acquiring a spectral distribution of interference light in a time-divisional manner. For non-limiting specific examples of spectral domain OCT, see U.S. Patent No. 9,295,386. For non-limiting specific examples of swept source OCT, see U.S. Patent No. 11,013,400.

The user interface 128 is an interface for exchanging information between the ophthalmic apparatus 120 and a user. The user interface 128 includes, for example, an input interface and an output interface. The input interface is an interface for a user to input information into the ophthalmic apparatus 120, and examples thereof include a pointing device, a keyboard, a switch, an audio input device, and an image (video) input device. The output interface is an interface for outputting information from the ophthalmic apparatus 120, and examples thereof include a display, an audio output device, and a printer.

The ophthalmic apparatus 120 in the present example may include various elements that are not shown in the drawings. For example, the ophthalmic apparatus 120 may include any elements described in the above documents (U.S. Patent No. 9,295,386, U.S. Patent No. 11,013,400) or any elements described in any other documents related to OCT.

The processor 121 includes the controller (control circuit, control circuitry) 122, the measurement value calculator (measurement value calculation circuit, measurement value calculation circuitry) 123, and the risk information generator (risk information generation circuit, risk information generation circuitry) 124.

The controller 122 is configured to perform control related to the measurement value calculator 123, control related to the risk information generator 124, control related to the memory 125, control related to the OCT data acquisition unit 127, and control related to the user interface 128.

The control related to the measurement value calculator 123 may include, for example, turning on/off the operation of the measurement value calculator 123, control related to a parameter of the measurement value calculator 123, input of data into the measurement value calculator 123, and reception of data output from the measurement value calculator 123. The control related to the risk information generator 124 may be performed in the same manner.

The control related to the memory 125 may include, for example, a process of storing (saving) data in the memory 125, a process of retrieving data from the memory 125, and a process of processing or editing the data stored in the memory 125.

The control related to the OCT data acquisition unit 127 may include, for example, control of the light source, control of the optical scanner, control of the optical path length of the sample arm and/or the reference arm, control of the focus of the measurement light, control of the polarization state of the measurement light and/or the reference light, control of the amount (intensity) of the measurement light and/or the reference light, control of the photodetector, and control of a data acquisition system (DAS, DAQ) that is not shown in the drawings.

The control related to the user interface 128 may include, for example, control of the display and control of the graphical user interface (GUI).

The controller 122 may perform control of other elements of the ophthalmic apparatus 120. This control may be performed in the same manner as conventional methods (e.g., alignment control, focus control, tracking control, and control of other kinds).

The controller 122 inputs the OCT data of the fundus of the subject's eye generated by the OCT data acquisition unit 127 into the measurement value calculator 123. The measurement value calculator 123 is configured to calculate, based on the OCT data, a plurality of measurement values which are to be input into the calculation model 126 used for glaucoma screening of the subject's eye.

In the case where the calculation model 126 includes two or more calculation models, for example, the controller 122 sends information indicating the type of measurement value corresponding to the calculation model used for the glaucoma screening of the subject's eye, to the measurement value calculator 123. The information is referred to as measurement value type information herein. Based on the measurement value type information, the measurement value calculator 123 recognizes the type of the plurality of measurement values to be calculated, and calculates each of these measurement values from the OCT data of the fundus of the subject's eye.

As mentioned earlier, the calculation model 126 in some examples may be a linear equation that includes a linear combination of one or more optic nerve head parameters indicating retinal tissue thickness in the optic nerve head area and a linear combination of one or more macular parameters indicating retinal tissue thickness in the macular area. In the case where the calculation model 126 thus configured is used for the glaucoma screening of the subject's eye, the plurality of measurement values calculated by the measurement value calculator 123 include one or more optic nerve head measurement values respectively corresponding to the one or more optic nerve head parameters and one or more macular measurement values respectively corresponding to the one or more macular parameters.

When the calculation model 126 used for the glaucoma screening of the subject's eye is the first calculation model Y (Model 1), the measurement value calculator 123 calculates the following measurement values based on the OCT data of the fundus of the subject's eye: a measurement value corresponding to TSNITlower; a measurement value corresponding to cpRNFLqS; a measurement value corresponding to cpRNFLqlt; a measurement value corresponding to cpRNFLqln; a measurement value corresponding to mGCIPL_IT; a measurement value corresponding to log mGCIPL_STvsIT; a measurement value corresponding to log mGCIPL_ITvsIN; and a measurement value corresponding to mGCC_IN.

When the calculation model 126 used for the glaucoma screening of the subject's eye is the second calculation model Y (Model 2), the measurement value calculator 123 calculates the following measurement values based on the OCT data of the fundus of the subject's eye: a measurement value corresponding to TSNITlower; a measurement value corresponding to cpRNFLqS; a measurement value corresponding to cpRNFLqlt; a measurement value corresponding to cpRNFLqln; a measurement value corresponding to log_mGCIPL_STxvsITx; and the measurement value corresponding to log_mGCC_ITxvsINx.

When the calculation model 126 used for the glaucoma screening of the subject's eye is the third calculation model Y (Model 3), the measurement value calculator 123 calculates the following measurement values based on the OCT data of the fundus of the subject's eye: a measurement value corresponding to TSNITlower; a measurement value corresponding to cpRNFLqln; a measurement value corresponding to mGCC; a measurement value corresponding to log_mGCIPL_SNyvsINy; a measurement value corresponding to log_mGCIPL_STyvsITy, and a measurement value corresponding to log_mGCC_ITyvsINy.

Similarly, in the case where a different type of the calculation model 126 is employed, the measurement value calculator 123 calculates a measurement value corresponding to each of the plurality of parameters (plurality of variables) included in the calculation model 126 from the OCT data of the fundus of the subject's eye.

The risk information generator 124 generates glaucoma risk information for the subject's eye based on the plurality of measurement values calculated by the measurement value calculator 123 from the OCT data of the fundus of the subject's eye, and on the calculation model 126 used for the glaucoma screening of the subject's eye.

In some examples, the risk information generator 124 substitutes each measurement value calculated by the measurement value calculator 123 into the corresponding parameter (variable) of the calculation model 126 and calculates a value (e.g., glaucoma risk score) based on the calculation model 126. The glaucoma risk information generated and output by the risk information generator 124 in the present example includes, for example, either or both of the result of the calculation performed using the calculation model 126 (e.g., glaucoma risk score) and information obtained by applying a predetermined algorithm (procedure, calculation method) to the result of the calculation (e.g., information indicating the degree of glaucoma risk).

When the calculation model 126 used for the glaucoma screening of the subject's eye is the first calculation model Y (Model 1), the risk information generator 124 calculates a glaucoma risk score by substituting the plurality of measurement values into the plurality of parameters (the plurality of variables) of the first calculation model Y (Model 1) as follows: (1) the measurement value corresponding to TSNITlower is substituted into the variable TSNITlower; (2) the measurement value corresponding to cpRNFLqS is substituted into the variable cpRNFLqS; (3) the measurement value corresponding to cpRNFLqlt is substituted into the variable cpRNFLqlt; (4) the measurement value corresponding to cpRNFLqln is substituted into the variable cpRNFLqln; (5) the measurement value corresponding to mGCIPL_IT is substituted into the variable mGCIPL_IT; (6) the measurement value corresponding to log_mGCIPL_STvsIT is substituted into the variable log_mGCIPL_STvsIT; (7) the measurement value corresponding to log_mGCIPL_ITvsIN is substituted into the variable log_mGCIPL_ITvsIN; and (8) the measurement value corresponding to mGCC_IN is substituted into the variable mGCC_IN. Further, the risk information generator 124 in the present example can generate glaucoma risk information that includes the glaucoma risk score calculated in the above manner and/or information indicating the degree of glaucoma risk determined from the glaucoma risk score (e.g., high risk, medium risk, or low risk).

When the calculation model 126 used for the glaucoma screening of the subject's eye is the second calculation model Y (Model 2), the risk information generator 124 calculates a glaucoma risk score by substituting the plurality of measurement values into the plurality of parameters (the plurality of variables) of the second calculation model Y (Model 2) as follows: (1) the measurement value corresponding to TSNITlower is substituted into the variable TSNITlower; (2) the measurement value corresponding to cpRNFLqS is substituted into the variable cpRNFLqS; (3) the measurement value corresponding to cpRNFLqlt is substituted into the variable cpRNFLqlt; (4) the measurement value corresponding to cpRNFLqln is substituted into the variable cpRNFLqln; (5) the measurement value corresponding to log_mGCIPL_STxvsITx is substituted into the variable log mGCIPL_STxvsITx; and (6) the measurement value corresponding to log_mGCC_ITxvsINx is substituted into the variable log_mGCC_ITxvsINx. Further, the risk information generator 124 in the present example can generate glaucoma risk information that includes the glaucoma risk score calculated in the above manner and/or information indicating the degree of glaucoma risk determined from the glaucoma risk score (e.g., high risk, medium risk, or low risk).

When the calculation model 126 used for the glaucoma screening of the subject's eye is the third calculation model Y (Model 3), the risk information generator 124 calculates a glaucoma risk score by substituting the plurality of measurement values into the plurality of parameters (the plurality of variables) of the third calculation model Y (Model 3) as follows: (1) the measurement value corresponding to TSNITlower is substituted into the variable TSNITlower; (2) the measurement value corresponding to cpRNFLqIn is substituted into the variable cpRNFLqIn; (3) the measurement value corresponding to mGCC is substituted into the variable mGCC; (4) the measurement value corresponding to log_mGCIPL_SNyvsINy is substituted into the variable log_mGCIPL_SNyvsINy; (5) the measurement value corresponding to log_mGCIPL_STyvsITy is substituted into the variable log_mGCIPL_STyvsITy; and (6) the measurement value corresponding to log_mGCC_ITyvsINy is substituted into the variable log_mGCC_ITyvsINy. Further, the risk information generator 124 in the present example can generate glaucoma risk information that includes the glaucoma risk score calculated in the above manner and/or information indicating the degree of glaucoma risk determined from the glaucoma risk score (e.g., high risk, medium risk, or low risk).

Similarly, in the case where a different type of the calculation model 126 is used, the risk information generator 124 can substitute the plurality of measurement values calculated by the measurement value calculator 123 into the plurality of variables of the calculation model 126 used for the glaucoma screening of the subject's eye, thereby generating glaucoma risk information.

The controller 122 performs control to provide the glaucoma risk information generated by the risk information generator 124. In some examples, the controller 122 may display the glaucoma risk information on a display device of the user interface 128. In some examples, the controller 122 may control a communication device (not shown in the drawings) to transmit the glaucoma risk information to another device (e.g., an apparatus, a system, a recording medium).

When the calculation model 126 includes a plurality of calculation models and glaucoma screening of the subject's eye is performed using two or more of these calculation models, the measurement value calculator 123 calculates two or more measurement value groups corresponding to the two or more calculation models from the OCT data of the fundus of the subject's eye, and the risk information generator 124 generates glaucoma risk information based on each of the two or more measurement value groups. As a result, two or more pieces of glaucoma risk information respectively corresponding to the two or more calculation models used in the glaucoma screening of the subject's eye is obtained.

In some examples, the risk information generator 124 can provide the two or more pieces of glaucoma risk information.

In some examples, the risk information generator 124 may select one or more pieces of glaucoma risk information from the two or more pieces of glaucoma risk information, and provide the one or more pieces of glaucoma risk information selected.

In some examples, the risk information generator 124 may generate final glaucoma risk information based on the two or more pieces of glaucoma risk information, and provide the final glaucoma risk information (and the two or more pieces of glaucoma risk information).

In some examples, the risk information generator 124 may generate a final glaucoma risk score and/or final information indicating the degree of glaucoma risk based on two or more glaucoma risk scores. The process of generating the final glaucoma risk information from two or more pieces of glaucoma risk information may include, for example, any of the following processes: a process of calculating a statistic (e.g., average, weighted average, median, standard deviation, variance, etc.) of the two or more glaucoma risk scores; and a process of eliminating an outlier from the two or more glaucoma risk scores. The final information indicating the degree of glaucoma risk may be generated from the final glaucoma risk score, for example.

In some examples, the risk information generator 124 may generate final information indicating the degree of glaucoma risk based on a plurality of pieces of information each indicating the degree of glaucoma risk. This final information generation process may include, for example, any of the following processes: a process of selecting one piece of information from the plurality of pieces of information by a majority vote; a process of selecting one piece of information from the plurality of pieces of information by comparing the information acquired from the subject's eye (subject) in the current glaucoma screening with the plurality of pieces of information; and a process of selecting one piece of information from the plurality of pieces of information by comparing information acquired from a past examination of the subject's eye (subject) with the plurality of pieces of information.

As described above, the ophthalmic apparatus 120 according to the present aspect can provide a high-quality glaucoma identification technique suitable for mass screening by performing glaucoma risk evaluation of the subject's eye using the glaucoma screening method described in the embodiments above.

< Program and recording medium >

Next, described are programs and recording media of non-limiting aspect examples of the glaucoma screening method according to the embodiments described above.

The program according to the present aspect is a novel program to be executed by a computer. The computer includes a processor and a memory. The processor may have a configuration same as or similar to that of the processor 121 of the ophthalmic apparatus 120 shown in FIG. 12 as an example. In other words, the processor according to the present aspect may include a processor that functions as the controller 122, a processor that functions as the measurement value calculator 123, and a processor that functions as the risk information generator 124. Furthermore, the memory according to the present aspect may retain a calculation model (the calculation model 126), like the memory 125 of the ophthalmic apparatus 120.

The program according to the present aspect is configured to cause the processor to execute a procedure including a series of steps described below.

In the first step, the processor executes control to store a calculation model in the memory in accordance with the program of the present aspect. The calculation model may be any of the calculation models according to the embodiments above. The calculation model may be, for example, a linear equation that includes a linear combination of one or more optic nerve head parameters indicating retinal tissue thickness in an optic nerve head area and a linear combination of one or more macular parameters indicating retinal tissue thickness in a macular area.

In the second step, the processor executes control to receive OCT data of the fundus of the subject's eye (OCT data reception control) in accordance with the program of the present aspect.

In some examples, the OCT data reception control may include a process of acquiring OCT data acquired from the fundus of the subject's eye by another ophthalmic apparatus (ophthalmic OCT apparatus) that includes an OCT scanner, from any of the ophthalmic OCT apparatus, a medical image archiving system, a recording medium, and the like.

In some examples, the computer may be an element of an ophthalmic apparatus (for example, the ophthalmic apparatus 120). The ophthalmic apparatus may include an OCT data acquisition unit (for example, the OCT data acquisition unit 127). In this case, the OCT data reception control may include control for causing the OCT data acquisition unit to perform OCT scanning on the fundus of the subject's eye, and control for causing the OCT data acquisition unit to generate OCT data based on data acquired through the OCT scanning.

To summarize, the OCT data reception control executed in the second step may include either the control for the processor to receive OCT data generated by another ophthalmic apparatus from outside the computer, or the control for the processor to receive OCT data generated by the ophthalmic apparatus itself that includes the computer.

In the third step, the processor executes processing of calculating a plurality of measurement values from the OCT data acquired in the second step (measurement value calculation processing) in accordance with the program of the present aspect.

In some examples, the measurement value calculation processing calculates a plurality of measurement values corresponding to the calculation model stored in the memory in the first step, based on the OCT data acquired in the second step. In the case where the calculation model is a linear equation that includes a linear combination of one or more optic nerve head parameters indicating retinal tissue thickness in the optic nerve head area and a linear combination of one or more macular parameters indicating retinal tissue thickness in the macular area, the measurement value calculation processing includes processing of calculating, based on the OCT data acquired in the second step, a plurality of measurement values that include one or more optic nerve head measurement values respectively corresponding to the one or more optic nerve head parameters and one or more macular measurement values respectively corresponding to the one or more macular parameters.

In the fourth step, the processor executes processing of inputting the plurality of measurement values calculated in the third step into the calculation model stored in the memory in the first step in accordance with the program of the present aspect.

In case where the calculation model stored in the memory in the first step is a linear equation that includes a linear combination of one or more optic nerve head parameters indicating retinal tissue thickness in the optic nerve head area and a linear combination of one or more macular parameters indicating retinal tissue thickness in the macular area, and also in case where a plurality of measurement values including one or more optic nerve head measurement values respectively corresponding to the one or more optic nerve head parameters and one or more macular measurement values respectively corresponding to the one or more macular parameters is calculated in the third step, then in the fourth step, each optic nerve head measurement value and each macular measurement value are input respectively into the corresponding optic nerve head parameter (variable) and the corresponding macular parameter (variable) of the calculation model. As a result, a glaucoma risk score of the subject's eye is derived. Then, the processor can generate glaucoma risk information from this glaucoma risk score.

In the fifth step, the processor executes processing of providing the glaucoma risk information generated in the fourth step in accordance with the program of the present aspect. In other words, the fifth step executes processing of providing a calculation result output from the calculation model in response to the input of the plurality of measurement values calculated in the third step.

In this way, the program according to the present aspect performs glaucoma risk evaluation of the subject's eye using the glaucoma screening method according to the embodiments above. Therefore, the program according to the present aspect is capable of providing a high-quality glaucoma identification technique suitable for mass screening.

The program according to the present aspect may be recorded on a recording medium. In other words, the recording medium according to the present aspect is a recording medium on which the program according to the present aspect is recorded. The recording medium according to the present aspect is a computer-readable non-transitory recording medium. The computer-readable non-transitory recording medium according to the present aspect may be a recording medium of any form, such as a magnetic disk, an optical disk, a magneto-optical disk, or a semiconductor memory.

Any matters or items related to the glaucoma screening method according to any of the embodiments, and/or any matters or items related to the ophthalmic apparatus according to any of the embodiments, may be combined at least in part with the program according to any of the aspect examples. Further, any known or existing techniques or technologies may also be combined with the program according to any of the aspect examples.

Likewise, any matters or items related to the glaucoma screening method according to any of the embodiments, and/or any matters or items related to the ophthalmic apparatus according to any of the embodiments, may be combined at least in part with the recording medium according to any of the aspect examples. Further, any known or existing techniques or technologies may also be combined with the recording medium according to any of the aspect examples.

< Some non-limiting features >

This section describes some non-limiting features of the various embodiments detailed in the present disclosure. Note that the features of the embodiments are not limited to those described in this section.

The first aspect example of a glaucoma screening method according to some embodiments includes a calculation model preparation step, an OCT data generation step, a measurement value calculation step, a measurement value input step, and a calculation result provision step.

In the calculation model preparation step, a calculation model is prepared that includes a linear combination of one or more optic nerve head parameters indicating retinal tissue thickness in an optic nerve head area and a linear combination of one or more macular parameters indicating retinal tissue thickness in a macular area.

In the OCT data generation step, optical coherence tomography (OCT) scanning is applied to a fundus of a subject's eye to generate OCT data.

In the measurement value calculation step, a plurality of measurement values is calculated based on the OCT data generated in the OCT data generation step. Here, the plurality of measurement values calculated includes one or more optic nerve head measurement values respectively corresponding to the one or more optic nerve head parameters of the calculation model prepared in the calculation model preparation step, and one or more macular measurement values respectively corresponding to the one or more macular parameters of the calculation model prepared in the calculation model preparation step.

In the measurement value input step, the plurality of measurement values calculated in the measurement value calculation step is input into the calculation model prepared in the calculation model preparation step.

In the calculation result provision step, a calculation result output from the calculation model in response to the input of the plurality of measurement values into the calculation model executed in the measurement value input step is provided.

The second aspect example of the glaucoma screening method according to some embodiments includes the following non-limiting feature in addition to the non-limiting features of the first aspect example. The one or more optic nerve head parameters of the calculation model prepared in the calculation model preparation step include optic nerve head parameters indicating retinal nerve fiber layer thickness in the optic nerve head area.

The third aspect example of the glaucoma screening method according to some embodiments includes the following non-limiting feature in addition to the non-limiting features of the second aspect example. The calculation model prepared in the calculation model preparation step includes a linear combination of two or more optic nerve head parameters. The two or more optic nerve head parameters include a first optic nerve head parameter (TSNITlower) indicating a layer thickness value corresponding to a lower peak of two peaks of a double hump of a TSNIT plot showing retinal nerve fiber layer thickness distribution in a predetermined circumpapillary circular region. Furthermore, the two or more optic nerve head parameters include a second optic nerve head parameter (cpRNFLqIn) indicating an average value of retinal nerve fiber layer thickness in an arc-shaped region of the circumpapillary circular region corresponding to a range of angle from 225 to 285 degrees, the angle being defined by setting a temporal direction to be zero and a rotation direction from the temporal direction to a superior direction to be a positive direction.

The fourth aspect example of the glaucoma screening method according to some embodiments includes the following non-limiting feature in addition to the non-limiting features of the third aspect example. The calculation model prepared in the calculation model preparation step includes a linear combination of four optic nerve head parameters. The four optic nerve head parameters include a third optic nerve head parameter (cpRNFLqS) and a fourth optic nerve head parameter (cpRNFLqIt) in addition to the first optic nerve head parameter (TSNITlower) and the second optic nerve head parameter (cpRNFLqIn). The third optic nerve head parameter (cpRNFLqS) is an optic nerve head parameter that indicates an average value of retinal nerve fiber layer thickness in an arc-shaped region of the circumpapillary circular region corresponding to a range of angle, according to the definition, from 45 to 135 degrees. The fourth optic nerve head parameter (cpRNFLqIt) is an optic nerve head parameter that indicates an average value of retinal nerve fiber layer thickness in an arc-shaped region of the circumpapillary circular region corresponding to a range of angle, according to the definition, from 285 to 315 degrees.

The fifth aspect example of the glaucoma screening method according to some embodiments includes the following non-limiting feature in addition to the non-limiting features of any of the first to fourth aspect examples. The calculation model prepared in the calculation model preparation step includes a linear combination of two or more macular parameters. The two or more macular parameters include a first macular parameter based on complex tissue thickness of a ganglion cell layer and an inner plexiform layer (mGCIPL) in the macular area, and a second macular parameter based on retinal ganglion cell complex (mGCC) thickness in the macular area.

The sixth aspect example of the glaucoma screening method according to some embodiments includes the following non-limiting feature in addition to the non-limiting features of the fifth aspect example. The first macular parameter (parameter related to mGCIPL) includes a layer thickness value parameter (mGCIPL_IT) indicating a value of the complex tissue (mGCIPL) thickness.

The seventh aspect example of the glaucoma screening method according to some embodiments includes the following non-limiting feature in addition to the non-limiting features of the fifth aspect example. The first macular parameter (parameter related to mGCIPL) includes a comparison value parameter (log_mGCIPL_STvsIT and log_mGCIPL_ITvsIN; log_mGCIPL_STxvsITx; log_mGCIPL_SNyvsINy and log_mGCIPL_STyvsITy) indicating a comparison value between first complex tissue thickness in a first subarea of the macular area and second complex tissue thickness in a second subarea.

The eighth aspect example of the glaucoma screening method according to some embodiments includes the following non-limiting feature in addition to the non-limiting features of the seventh aspect example. The comparison value indicated by the comparison value parameter included in the first macular parameter (parameter related to mGCIPL) is calculated by performing logarithm calculation in which an antilogarithm includes difference between the first complex tissue thickness in the first subarea of the macular area and the second complex tissue thickness in the second subarea.

The ninth aspect example of the glaucoma screening method according to some embodiments includes the following non-limiting feature in addition to the non-limiting features of any of the fifth to eighth aspect examples. The second macular parameter (parameter related to mGCC) includes a layer thickness value parameter (mGCC_IN; mGCC) indicating a value of the retinal ganglion cell complex (mGCC) thickness.

The tenth aspect example of the glaucoma screening method according to some embodiments includes the following non-limiting feature in addition to the non-limiting features of any of the fifth to eighth aspect examples. The second macular parameter (parameter related to mGCC) includes a comparison value parameter (log_mGCC_ITxvsINx; log_mGCC_ITyvsINy) indicating a comparison value between first retinal ganglion cell complex thickness in a third subarea of the macular area and second retinal ganglion cell complex thickness in a fourth subarea.

The eleventh aspect example of the glaucoma screening method according to some embodiments includes the following non-limiting feature in addition to the non-limiting features of the tenth aspect example. The comparison value indicated by the comparison value parameter included in the second macular parameter (parameter related to mGCC) is calculated by performing logarithm calculation in which an antilogarithm includes difference between the first retinal ganglion cell complex thickness in the third subarea of the macular area and the second retinal ganglion cell complex thickness in the fourth subarea.

The twelfth aspect example of the glaucoma screening method according to some embodiments includes the following non-limiting feature in addition to the non-limiting features of any of the first to eleventh aspect examples. In the calculation model preparation step, the calculation model is created based on a development dataset collected from a first group, a first validation dataset collected from a second group different from the first group, and a second validation dataset collected from a subgroup of the first group determined (judged) to be suspected of glaucoma.

The thirteenth aspect example of the glaucoma screening method according to some embodiments includes the following non-limiting feature in addition to the non-limiting features of the twelfth aspect example. The development dataset includes fundus photographs acquired using digital fundus photography, fundus OCT data acquired using optical coherence tomography, and visual field data acquired using a visual field test.

The fourteenth aspect example of the glaucoma screening method according to some embodiments includes the following non-limiting feature in addition to the non-limiting features of the twelfth or thirteenth aspect example. In the calculation model preparation step, the first validation dataset includes fundus OCT data acquired using optical coherence tomography and is used in positive predictive value validation.

The fifteenth aspect example of the glaucoma screening method according to some embodiments includes the following non-limiting feature in addition to the non-limiting features of any of the twelfth to fourteenth aspect examples. In the calculation model preparation step, the second validation dataset includes visual field data acquired using a visual field test and is used in sensitivity validation and specificity validation.

Any of the first to fifteenth aspect examples of the glaucoma screening method according to some embodiments may be combined, at least in part, with any of the matters or items described or suggested in the present disclosure.

The first aspect example of an ophthalmic apparatus according to some embodiments includes a memory, an OCT data acquisition unit, a measurement value calculator, and a risk information generator.

The memory is configured to retain a calculation model that is created in advance. The calculation model includes a linear combination of one or more optic nerve head parameters indicating retinal tissue thickness in an optic nerve head area and a linear combination of one or more macular parameters indicating retinal tissue thickness in a macular area. The memory 125 of the ophthalmic apparatus 120 shown in FIG. 12 is an example of the memory according to the present embodiment.

The OCT data acquisition unit is configured to acquire OCT data of a fundus of a subject's eye. The OCT data acquisition unit 127 of the ophthalmic apparatus 120 shown in FIG. 12 is an example of the OCT data acquisition unit according to the present embodiment.

The measurement value calculator is configured to calculate, based on the OCT data acquired by the OCT data acquisition unit, a plurality of measurement values including one or more optic nerve head measurement values respectively corresponding to the one or more optic nerve head parameters and one or more macular measurement values respectively corresponding to the one or more macular parameters in the calculation model stored in the memory. The measurement value calculator 123 of the ophthalmic apparatus 120 shown in FIG. 12 is an example of the measurement value calculator according to the present embodiment.

The risk information generator is configured to generate glaucoma risk information of the subject's eye based on the calculation model stored in the memory and the plurality of measurement values calculated by the measurement value calculator. The risk information generator 124 of the ophthalmic apparatus 120 shown in FIG. 12 is an example of the risk information generator according to the present embodiment.

The second aspect example of the ophthalmic apparatus according to some embodiments includes the following non-limiting feature in addition to the non-limiting features of the first aspect example. The one or more optic nerve head parameters of the calculation model stored in the memory include optic nerve head parameters indicating retinal nerve fiber layer thickness in the optic nerve head area.

The third aspect example of the ophthalmic apparatus according to some embodiments includes the following non-limiting feature in addition to the non-limiting features of the second aspect example. The calculation model stored in the memory includes a linear combination of two or more optic nerve head parameters. The two or more optic nerve head parameters include a first optic nerve head parameter (TSNITlower) indicating a layer thickness value corresponding to a lower peak of two peaks of a double hump of a TSNIT plot showing retinal nerve fiber layer thickness distribution in a predetermined circumpapillary circular region. Furthermore, the two or more optic nerve head parameters include a second optic nerve head parameter (cpRNFLqIn) indicating an average value of retinal nerve fiber layer thickness in an arc-shaped region of the circumpapillary circular region corresponding to a range of angle from 225 to 285 degrees, the angle being defined by setting a temporal direction to be zero and a rotation direction from the temporal direction to a superior direction to be a positive direction.

The fourth aspect example of the ophthalmic apparatus according to some embodiments includes the following non-limiting feature in addition to the non-limiting features of the third aspect example. The calculation model stored in the memory includes a linear combination of four optic nerve head parameters. The four optic nerve head parameters include, in addition to the first optic nerve head parameter (TSNITlower) and the second optic nerve head parameter (cpRNFLqIn), a third optic nerve head parameter (cpRNFLqS) and a fourth optic nerve head parameter (cpRNFLqIt). The third optic nerve head parameter (cpRNFLqS) is an optic nerve head parameter that indicates an average value of retinal nerve fiber layer thickness in an arc-shaped region of the circumpapillary circular region corresponding to a range of angle, according to the definition, from 45 to 135 degrees. The fourth optic nerve head parameter (cpRNFLqIt) is an optic nerve head parameter that indicates an average value of retinal nerve fiber layer thickness in an arc-shaped region of the circumpapillary circular region corresponding to a range of angle, according to the definition, from 285 to 315 degrees.

The fifth aspect example of the ophthalmic apparatus according to some embodiments includes the following non-limiting feature in addition to the non-limiting features of any of the first to fourth aspect examples. The calculation model stored in the memory includes a linear combination of two or more macular parameters. The two or more macular parameters include a first macular parameter based on complex tissue thickness of a ganglion cell layer and an inner plexiform layer (mGCIPL) in the macular area, and a second macular parameter based on retinal ganglion cell complex (mGCC) thickness in the macular area.

The sixth aspect example of the ophthalmic apparatus according to some embodiments includes the following non-limiting feature in addition to the non-limiting features of the fifth aspect example. The first macular parameter (parameter related to mGCIPL) includes a layer thickness value parameter (mGCIPL_IT) indicating a value of the complex tissue (mGCIPL) thickness.

The seventh aspect example of the ophthalmic apparatus according to some embodiments includes the following non-limiting feature in addition to the non-limiting features of the fifth aspect example. The first macular parameter (parameter related to mGCIPL) includes a comparison value parameter (log_mGCIPL_STvsIT and log_mGCIPL_ITvsIN; log_mGCIPL_STxvsITx; log_mGCIPL_SNyvsINy and log_mGCIPL_STyvsITy) indicating a comparison value between first complex tissue thickness in a first subarea of the macular area and second complex tissue thickness in a second subarea.

The eighth aspect example of the ophthalmic apparatus according to some embodiments includes the following non-limiting feature in addition to the non-limiting features of the seventh aspect example. The comparison value indicated by the comparison value parameter included in the first macular parameter (parameter related to mGCIPL) is calculated by performing logarithm calculation in which an antilogarithm includes difference between the first complex tissue thickness in the first subarea of the macular area and the second complex tissue thickness in the second subarea.

The ninth aspect example of the ophthalmic apparatus according to some embodiments includes the following non-limiting feature in addition to the non-limiting features of any of the fifth to eighth aspect examples. The second macular parameter (parameter related to mGCC) includes a layer thickness value parameter (mGCC_IN; mGCC) indicating a value of the retinal ganglion cell complex (mGCC) thickness.

The tenth aspect example of the ophthalmic apparatus according to some embodiments includes the following non-limiting feature in addition to the non-limiting features of any of the fifth to eighth aspect examples. The second macular parameter (parameter related to mGCC) includes a comparison value parameter (log_mGCC_ITxvsINx; log_mGCC_ITyvsINy) indicating a comparison value between first retinal ganglion cell complex thickness in a third subarea of the macular area and second retinal ganglion cell complex thickness in a fourth subarea.

The eleventh aspect example of the ophthalmic apparatus according to some embodiments includes the following non-limiting feature in addition to the non-limiting features of the tenth aspect example. The comparison value indicated by the comparison value parameter included in the second macular parameter (parameter related to mGCC) is calculated by performing logarithm calculation in which an antilogarithm includes difference between the first retinal ganglion cell complex thickness in the third subarea of the macular area and the second retinal ganglion cell complex thickness in the fourth subarea.

The twelfth aspect example of the ophthalmic apparatus according to some embodiments includes the following non-limiting feature in addition to the non-limiting features of any of the first to eleventh aspect examples. The calculation model according to the present aspect example is created by a calculation model creation apparatus. The calculation model creation apparatus may be an element of the ophthalmic apparatus according to the present aspect example, or may be an apparatus separate from the ophthalmic apparatus according to the present aspect example.

To create the calculation model according to the present aspect example, a development dataset, a first validation dataset, and a second validation dataset are prepared.

The development dataset is a dataset collected from a first group. More specifically, the development dataset is a dataset collected by applying one or more predetermined medical examinations or tests (including ophthalmic examinations or tests) to each subject included in the first group.

The first validation dataset is a dataset collected from a second group different from the first group corresponding to the development dataset. More specifically, the first validation dataset is a dataset collected by applying one or more predetermined medical examinations or tests (including ophthalmic examinations or tests) to each subject included in the second group. The one or more medical examinations or tests performed for the first validation dataset and the one or more medical examinations or tests performed for the development dataset may have at least one common examination or test, or may not have any common examination or test.

The second validation dataset is a dataset collected from a subgroup of the first group, corresponding to the development dataset, that has been determined to be suspected of glaucoma. The subgroup is a group of subjects who have been judged (diagnosed) to be suspected of having glaucoma among the plurality of subjects belonging to the first group that participated in the creation of the development dataset as targets. It is clear from the definition that this group is a part (subset) of the first group.

The development dataset, the first validation dataset, and the second validation dataset are input into the calculation model creation apparatus. The calculation model creation apparatus creates a calculation model based on these datasets and a predetermined algorithm.

The algorithm used to create the calculation model may include a machine learning algorithm. This machine learning method is typically supervised learning. However, the machine learning method is not limited to supervised learning. In some examples, in addition to or instead of supervised learning, any known method such as unsupervised learning, reinforcement learning, semi-supervised learning, transduction, or multitask learning may be used alone or in combination for the machine learning for constructing the calculation model.

The method used to construct the calculation model is not limited to the examples presented here, and may include any known method such as support vector machines, Bayesian classifiers, boosting, k-means clustering, kernel density estimation, principal component analysis, independent component analysis, self-organizing maps, random forests, and generative adversarial networks (GANs).

The thirteenth aspect example of the ophthalmic apparatus according to some embodiments includes the following non-limiting feature in addition to the non-limiting features of the twelfth aspect example. The development dataset input into the calculation model creation apparatus includes fundus photographs acquired using digital fundus photography, fundus OCT data acquired using optical coherence tomography, and visual field data acquired using a visual field test.

The fourteenth aspect example of the ophthalmic apparatus according to some embodiments includes the following non-limiting feature in addition to the non-limiting features of the twelfth or thirteenth aspect example. The first validation dataset input into the calculation model creation apparatus includes fundus OCT data acquired using optical coherence tomography. The calculation model creation apparatus performs positive predictive value validation based on the first validation dataset input.

The fifteenth aspect example of the ophthalmic apparatus according to some embodiments includes the following non-limiting feature in addition to the non-limiting features of any of the twelfth to fourteenth aspect examples. The second validation dataset input into the calculation model creation apparatus includes visual field data acquired using a visual field test. The calculation model creation apparatus performs sensitivity validation and specificity validation based on the second validation dataset input.

Any of the first to fifteenth aspect examples of the ophthalmic apparatus according to some embodiments may be combined, at least in part, with any of the matters or items described or suggested in the present disclosure.

The first aspect example of a program according to some embodiments is a program executed by a computer including a processor and a memory. The program is configured to cause the processor to execute calculation model storage control, OCT data reception control, measurement value calculation processing, measurement value input processing, and calculation result provision processing.

Through the calculation model storage control, the program causes the processor to execute processing of storing, into the memory, a calculation model that includes a linear combination of one or more optic nerve head parameters indicating retinal tissue thickness in an optic nerve head area and a linear combination of one or more macular parameters indicating retinal tissue thickness in a macular area.

Through the OCT data reception control, the program causes the processor to execute processing of receiving optical coherence tomography (OCT) data of a fundus of a subject's eye.

In the measurement value calculation processing, the program causes the processor to execute processing of calculating, based on the OCT data received in the OCT data reception processing, a plurality of measurement values including one or more optic nerve head measurement values respectively corresponding to the one or more optic nerve head parameters and one or more macular measurement values respectively corresponding to the one or more macular parameters in the calculation model stored in the memory through the calculation model storage control.

In the measurement value input processing, the program causes the processor to execute processing of inputting the plurality of measurement values calculated in the measurement value calculation processing into the calculation model stored in the memory through the calculation model storage control.

In the calculation result provision processing, the program causes the processor to execute processing of providing a calculation result output from the calculation model in response to the input of the plurality of measurement values into the calculation model executed through the measurement value input processing.

The first aspect example of a recording medium according to some embodiments is a computer-readable non-transitory recording medium storing the program according to the first aspect example.

The second aspect example of the program according to some embodiments includes the following non-limiting feature in addition to the non-limiting features of the first aspect example. The one or more optic nerve head parameters of the calculation model stored in the memory through the calculation model storage control, include optic nerve head parameters indicating retinal nerve fiber layer thickness in the optic nerve head area.

The second aspect example of the recording medium according to some embodiments is a computer-readable non-transitory recording medium storing the program according to the second aspect example.

The third aspect example of the program according to some embodiments includes the following non-limiting feature in addition to the non-limiting features of the second aspect example. The calculation model stored in the memory through the calculation model storage control includes a linear combination of two or more optic nerve head parameters. The two or more optic nerve head parameters include a first optic nerve head parameter (TSNITlower) indicating a layer thickness value corresponding to a lower peak of two peaks of a double hump of a TSNIT plot showing retinal nerve fiber layer thickness distribution in a predetermined circumpapillary circular region. Additionally, the two or more optic nerve head parameters include a second optic nerve head parameter (cpRNFLqIn) indicating an average value of retinal nerve fiber layer thickness in an arc-shaped region of the circumpapillary circular region corresponding to a range of angle from 225 to 285 degrees, the angle being defined by setting a temporal direction to be zero and a rotation direction from the temporal direction to a superior direction to be a positive direction.

The third aspect example of the recording medium according to some embodiments is a computer-readable non-transitory recording medium storing the program according to the third aspect example.

The fourth aspect example of the program according to some embodiments includes the following non-limiting feature in addition to the non-limiting features of the third aspect example. The calculation model stored in the memory through the calculation model storage control includes a linear combination of four optic nerve head parameters. The four optic nerve head parameters include the first optic nerve head parameter (TSNITlower), the second optic nerve head parameter (cpRNFLqIn), a third optic nerve head parameter (cpRNFLqS), and a fourth optic nerve head parameter (cpRNFLqIt). The third optic nerve head parameter (cpRNFLqS) is an optic nerve head parameter that indicates an average value of retinal nerve fiber layer thickness in an arc-shaped region of the circumpapillary circular region corresponding to a range of angle, according to the definition, from 45 to 135 degrees. The fourth optic nerve head parameter (cpRNFLqIt) is an optic nerve head parameter that indicates an average value of retinal nerve fiber layer thickness in an arc-shaped region of the circumpapillary circular region corresponding to a range of angle, according to the definition, from 285 to 315 degrees.

The fourth aspect example of the recording medium according to some embodiments is a computer-readable non-transitory recording medium storing the program according to the fourth aspect example.

The fifth aspect example of the program according to some embodiments includes the following non-limiting feature in addition to the non-limiting features of any of the first to fourth aspect examples. The calculation model stored in the memory through the calculation model storage control includes a linear combination of two or more macular parameters. The two or more macular parameters include a first macular parameter based on complex tissue thickness of a ganglion cell layer and an inner plexiform layer (mGCIPL) in the macular area, and a second macular parameter based on retinal ganglion cell complex (mGCC) thickness in the macular area.

The fifth aspect example of the recording medium according to some embodiments is a computer-readable non-transitory recording medium storing the program according to the fifth aspect example.

The sixth aspect example of the program according to some embodiments includes the following non-limiting feature in addition to the non-limiting features of the fifth aspect example. The first macular parameter (parameter related to mGCIPL) includes a layer thickness value parameter (mGCIPL_IT) indicating a value of the complex tissue (mGCIPL) thickness.

The sixth aspect example of the recording medium according to some embodiments is a computer-readable non-transitory recording medium storing the program according to the sixth aspect example.

The seventh aspect example of the program according to some embodiments includes the following non-limiting feature in addition to the non-limiting features of the fifth aspect example. The first macular parameter (parameter related to mGCIPL) includes a comparison value parameter (log_mGCIPL_STvsIT and log_mGCIPL_ITvsIN; log_mGCIPL_STxvsITx; log_mGCIPL_SNyvsINy and log_mGCIPL_STyvsITy) indicating a comparison value between first complex tissue thickness in a first subarea of the macular area and second complex tissue thickness in a second subarea.

The seventh aspect example of the recording medium according to some embodiments is a computer-readable non-transitory recording medium storing the program according to the seventh aspect example.

The eighth aspect example of the program according to some embodiments includes the following non-limiting feature in addition to the non-limiting features of the seventh aspect example. The comparison value indicated by the comparison value parameter included in the first macular parameter (parameter related to mGCIPL) is calculated by performing logarithm calculation in which an antilogarithm includes difference between the first complex tissue thickness in the first subarea of the macular area and the second complex tissue thickness in the second subarea.

The eighth aspect example of the recording medium according to some embodiments is a computer-readable non-transitory recording medium storing the program according to the eighth aspect example.

The ninth aspect example of the program according to some embodiments includes the following non-limiting feature in addition to the non-limiting features of any of the fifth to eighth aspect examples. The second macular parameter (parameter related to mGCC) includes a layer thickness value parameter (mGCC_IN; mGCC) indicating a value of the retinal ganglion cell complex (mGCC) thickness.

The ninth aspect example of the recording medium according to some embodiments is a computer-readable non-transitory recording medium storing the program according to the ninth aspect example.

The tenth aspect example of the program according to some embodiments includes the following non-limiting feature in addition to the non-limiting features of any of the fifth to eighth aspect examples. The second macular parameter (parameter related to mGCC) includes a comparison value parameter (log_mGCC_ITxvsINx; log_mGCC_ITyvsINy) indicating a comparison value between first retinal ganglion cell complex thickness in a third subarea of the macular area and second retinal ganglion cell complex thickness in a fourth subarea.

The tenth aspect example of the recording medium according to some embodiments is a computer-readable non-transitory recording medium storing the program according to the tenth aspect example.

The eleventh aspect example of the program according to some embodiments includes the following non-limiting feature in addition to the non-limiting features of the tenth aspect example. The comparison value indicated by the comparison value parameter included in the second macular parameter (parameter related to mGCC) is calculated by performing logarithm calculation in which an antilogarithm includes difference between the first retinal ganglion cell complex thickness in the third subarea of the macular area and the second retinal ganglion cell complex thickness in the fourth subarea.

The eleventh aspect example of the recording medium according to some embodiments is a computer-readable non-transitory recording medium storing the program according to the eleventh aspect example.

The twelfth aspect example of the program according to some embodiments includes the following non-limiting feature in addition to the non-limiting features of any of the first to eleventh aspect examples. The program according to the present aspect example is configured to cause the processor to further execute calculation model creation processing for creating the calculation model stored in the memory through the calculation model storage control.

In the calculation model creation processing, the program causes the processor to execute processing of creating the calculation model based on a development dataset collected from a first group, a first validation dataset collected from a second group different from the first group, and a second validation dataset collected from a subgroup of the first group determined to be suspected of glaucoma.

The twelfth aspect example of the recording medium according to some embodiments is a computer-readable non-transitory recording medium storing the program according to the twelfth aspect example.

The thirteenth aspect example of the program according to some embodiments includes the following non-limiting feature in addition to the non-limiting features of the twelfth aspect example. The development dataset input into the processor includes fundus photographs acquired using digital fundus photography, fundus OCT data acquired using optical coherence tomography, and visual field data acquired using a visual field test.

The thirteenth aspect example of the recording medium according to some embodiments is a computer-readable non-transitory recording medium storing the program according to the thirteenth aspect example.

The fourteenth aspect example of the program according to some embodiments includes the following non-limiting feature in addition to the non-limiting features of the twelfth or thirteenth aspect example. The first validation dataset input into the processor includes fundus OCT data acquired using optical coherence tomography. The processor performs positive predictive value validation, in accordance with the program, based on the first validation dataset input.

The fourteenth aspect example of the recording medium according to some embodiments is a computer-readable non-transitory recording medium storing the program according to the fourteenth aspect example.

The fifteenth aspect example of the program according to some embodiments includes the following non-limiting feature in addition to the non-limiting features of any of the twelfth to fourteenth aspect examples. The second validation dataset input into the processor includes visual field data acquired using a visual field test. The processor performs sensitivity validation and specificity validation, in accordance with the program, based on the second validation dataset input.

The fifteenth aspect example of the recording medium according to some embodiments is a computer-readable non-transitory recording medium storing the program according to the fifteenth aspect example.

Any of the first to fifteenth aspect examples of the program according to some embodiments may be combined, at least in part, with any of the matters or items described or suggested in the present disclosure.

Any of the first to fifteenth aspect examples of the recording medium according to some embodiments may be combined, at least in part, with any of the matters or items described or suggested in the present disclosure.

The present disclosure presents some embodiments and some aspects thereof. These embodiments and aspects are merely non-limiting examples of the present invention. Therefore, it is possible to make any modifications (e.g., omissions, substitutions, additions, etc.) to the embodiments and aspects presented in the present disclosure within the scope of the gist of the present invention.

[EXPLANATION OF REFERENCE CHARACTERS]

| | |
|---|---|
| 120 | Ophthalmic apparatus |
| 121 | Processor |
| 122 | Controller |
| 123 | Measurement value calculator |
| 124 | Risk information generator |
| 125 | Memory |
| 126 | Calculation model |
| 127 | OCT data acquisition unit |
| 128 | User interface |

**Claims**

1. A method of glaucoma screening comprising:

preparing a calculation model that includes a linear combination of one or more optic nerve head parameters indicating retinal tissue thickness in an optic nerve head area and a linear combination of one or more macular parameters indicating retinal tissue thickness in a macular area;

generating OCT data by applying an optical coherence tomography scan to a fundus of a subject's eye;

calculating, based on the OCT data, a plurality of measurement values including one or more optic nerve head measurement values respectively corresponding to the one or more optic nerve head parameters and one or more macular measurement values respectively corresponding to the one or more macular parameters;

inputting the plurality of measurement values into the calculation model; and

providing a calculation result output from the calculation model in response to the inputting the plurality of measurement values.

2. The method of claim 1, wherein the one or more optic nerve head parameters include an optic nerve head parameter indicating retinal nerve fiber layer thickness in the optic nerve head area.

3. The method of claim 2, wherein the calculation model includes a linear combination of two or more optic nerve head parameters,

wherein the two or more optic nerve head parameters includes:

a first optic nerve head parameter indicating a layer thickness value corresponding to a lower peak of two peaks of a double hump of a TSNIT plot showing retinal nerve fiber layer thickness distribution in a predetermined circumpapillary circular region, and

a second optic nerve head parameter indicating an average value of retinal nerve fiber layer thickness in an arc-shaped region of the circular region corresponding to a range of angle from 225 to 285 degrees, the angle being defined by setting a temporal direction to be zero and a rotation direction from the temporal direction to a superior direction to be a positive direction.

4. The method of claim 3, wherein the calculation model includes a linear combination of four optic nerve head parameters,

wherein the four optic nerve head parameters are:

the first optic nerve head parameter,

the second optic nerve head parameter,

a third optic nerve head parameter indicating an average value of retinal nerve fiber layer thickness in an arc-shaped region of the circular region corresponding to a range of angle from 45 to 135 degrees, and

a fourth optic nerve head parameter indicating an average value of retinal nerve fiber layer thickness in an arc-shaped region of the circular region corresponding to a range of angle from 285 to 315 degrees.

5. The method of any of claims 1 to 4, wherein the calculation model includes a linear combination of two or more macular parameters,

wherein the two or more macular parameters include:

a first macular parameter based on complex tissue thickness of a ganglion cell layer and an inner plexiform layer in the macular area, and

a second macular parameter based on retinal ganglion cell complex thickness in the macular area.

6. The method of claim 5, wherein the first macular parameter includes a layer thickness value parameter indicating a value of the complex tissue thickness.

7. The method of claim 5, wherein the first macular parameter includes a comparison value parameter indicating a comparison value between first complex tissue thickness in a first subarea of the macular area and second complex tissue thickness in a second subarea.

8. The method of claim 7, wherein the comparison value is calculated by performing logarithm calculation in which an antilogarithm includes difference between the first complex tissue thickness and the second complex tissue thickness.

9. The method of claim 5, wherein the second macular parameter includes a layer thickness value parameter indicating a value of the retinal ganglion cell complex thickness.

10. The method of claim 5, wherein the second macular parameter includes a comparison value parameter indicating a comparison value between first retinal ganglion cell complex thickness in a third subarea of the macular area and second retinal ganglion cell complex thickness in a fourth subarea.

11. The method of claim 10, wherein the comparison value is calculated by performing logarithm calculation in which an antilogarithm includes difference between the first retinal ganglion cell complex thickness and the second retinal ganglion cell complex thickness.

12. The method of any of claims 1 to 4, wherein the preparing the calculation model includes creating the calculation model based on a development dataset collected from a first group, a first validation dataset collected from a second group different from the first group, and a second validation dataset collected from a subgroup of the first group determined to be suspected of glaucoma.

13. The method of claim 12, wherein the development dataset includes fundus photographs acquired using digital fundus photography, fundus OCT data acquired using optical coherence tomography, and visual field data acquired using a visual field test.

14. The method of claim 12, wherein the first validation dataset includes fundus OCT data acquired using optical coherence tomography and is used in positive predictive value validation.

15. The method of claim 12, wherein the second validation dataset includes visual field data acquired using a visual field test and is used in sensitivity validation and specificity validation.

16. An ophthalmic apparatus comprising:

a memory configured to retain a calculation model that is created in advance and includes a linear combination of one or more optic nerve head parameters indicating retinal tissue thickness in an optic nerve head area and a linear combination of one or more macular parameters indicating retinal tissue thickness in a macular area;
an optical coherence tomography (OCT) data acquisition unit configured to acquire OCT data of a fundus of a subject's eye;
a measurement value calculator configured to calculate, based on the OCT data, a plurality of measurement values including one or more optic nerve head measurement values respectively corresponding to the one or more optic nerve head parameters and one or more macular measurement values respectively corresponding to the one or more macular parameters; and
a risk information generator configured to generate glaucoma risk information of the subject's eye based on the calculation model and the plurality of measurement values.

17. A program executed by a computer including a processor and a memory, the program configured to cause the processor to execute:

control of storing, in the memory, a calculation model that includes a linear combination of one or more optic nerve head parameters indicating retinal tissue thickness in an optic nerve head area and a linear combination of one or more macular parameters indicating retinal tissue thickness in a macular area;
control of receiving optical coherence tomography (OCT) data of a fundus of a subject's eye;
processing of calculating, based on the OCT data, a plurality of measurement values including one or more optic nerve head measurement values respectively corresponding to the one or more optic nerve head parameters and one or more macular measurement values respectively corresponding to the one or more macular parameters;
processing of inputting the plurality of measurement values into the calculation model; and
processing of providing a calculation result output from the calculation model in response to the inputting the plurality of measurement values.

18. A computer-readable non-transitory recording medium storing the program of claim 17.

Fig. 1

```
                    ╭─────────────╮
                    │    Start     │
                    ╰──────┬──────╯
                           │
  S11                      ▼
  ┌────────────────────────────────────────────────┐
  │ Create glaucoma risk score calculation model    │
  │ including ONH parameters and macular parameters  │
  └────────────────────────┬───────────────────────┘
                           │
  S12                      ▼
  ┌────────────────────────────────────────────────┐
  │         Generate OCT data of eye fundus          │
  └────────────────────────┬───────────────────────┘
                           │
  S13                      ▼
  ┌────────────────────────────────────────────────┐
  │     Calculate ONH measurement values and         │
  │  macular measurement values from OCT data         │
  └────────────────────────┬───────────────────────┘
                           │
  S14                      ▼
  ┌────────────────────────────────────────────────┐
  │   Input measurement values into calculation model │
  └────────────────────────┬───────────────────────┘
                           │
  S15                      ▼
  ┌────────────────────────────────────────────────┐
  │          Provide glaucoma risk score             │
  │          output from calculation model            │
  └────────────────────────┬───────────────────────┘
                           │
                           ▼
                    ╭─────────────╮
                    │     End      │
                    ╰─────────────╯
```

EP 4 555 918 A1

Fig. 2

Start

S21
Create calculation model

S22
Generate OCT data of eye fundus

S23
Calculate ONH measurement values and
macular measurement values from OCT data

S24
Input measurement values into calculation model

S25
Regulate coefficients of calculation model
based on output from calculation model

S26
Input measurement values into
updated calculation model

S27
Provide glaucoma risk score
output from updated calculation model

End

EP 4 555 918 A1

Fig. 3

```
                        ┌──────────┐
                        │  Start   │
                        └──────────┘
                              │
                              ▼
  S31
    ┌─────────────────────────────────────────┐
    │       Create development dataset         │
    └─────────────────────────────────────────┘
                              │
                              ▼
  S32
    ┌─────────────────────────────────────────┐
    │  Select eligible cases and eligible controls │
    └─────────────────────────────────────────┘
                              │
                              ▼
  S33
    ┌─────────────────────────────────────────┐
    │  Perform background condition matching   │
    │         between cases and controls       │
    └─────────────────────────────────────────┘
                              │
                              ▼
  S34
    ┌─────────────────────────────────────────┐
    │    Perform calculation model development │
    └─────────────────────────────────────────┘
                              │
                              ▼
  S35
    ┌─────────────────────────────────────────┐
    │        Create validation dataset         │
    └─────────────────────────────────────────┘
```

S36

Perform calculation model validation regarding PPV

S37

Perform calculation model validation regarding sensitivity and specificity

End

EP 4 555 918 A1

Fig. 4A

EP 4 555 918 A1

Fig. 4B

Model 1

Model 2

Model 3

EP 4 555 918 A1

EP 4 555 918 A1

Fig. 4C

Fig. 5

| | Cases (284 Eyes of 191 Participants) | Controls (284 Eyes of 277 Participants) | P Value |
|---|---|---|---|
| Gender | | | 1.00 |
|    Female | 33 (11.6) | 33 (11.6) | |
|    Male | 251 (88.4) | 251 (88.4) | |
| Age (years) | $56.3 \pm 9.2$ | $56.2 \pm 9.0$ | .96 |
| SD-OCT measured thickness (µm) | | | |
|    cpRNFL (10 × 10 grids) | $73.3 \pm 15.4$ | $99.3 \pm 10.1$ | <.01 |
|    cpRNFLqS | $88.2 \pm 24.3)$ | $123.9 \pm 16.5$ | <.01 |
|    cpRNFLqIt | $91.5 \pm 35.8$ | $142.7 \pm 23.6$ | <.01 |
|    cpRNFLqIn | $82.4 \pm 25.7$ | $119.1 \pm 19.3$ | <.01 |
|    TSNITlower | $94.8 \pm 23.3$ | $140.4 \pm 17.8$ | <.01 |
|    mRNFL (10 × 10 grids) | $32.8 \pm 5.2$ | $37.6 \pm 4.3$ | <.01 |
|    mGCIPL (10 × 10 grids) | $54.7 \pm 7.1$ | $65.4 \pm 5.2$ | <.01 |
|    mGCIPL_IT | $52.0 \pm 9.0$ | $65.3 \pm 5.3$ | <.01 |
|    log_mGCIPL_STvsIT | $1.9 \pm 0.8$ | $0.9 \pm 0.6$ | <.01 |
|    log_mGCIPL_ITvsIN | $1.7 \pm 0.7$ | $1.1 \pm 0.6$ | <.01 |
|    log_mGCIPL_STxvsITx | $2.1 \pm 1.0$ | $1.1 \pm 0.6$ | <.01 |
|    log_mGCIPL_SNyvsINy | $2.2 \pm 1.0$ | $1.1 \pm 0.6$ | <.01 |
|    log_mGCIPL_STyvsITy | $1.7 \pm 0.8$ | $1.2 \pm 0.6$ | <.01 |
|    mGCC (10 × 10 grids) | $87.4 \pm 10.4$ | $103.0 \pm 8.0$ | <.01 |
|    mGCC_IN | $95.7 \pm 15.6$ | $114.8 \pm 10.4$ | <.01 |
|    log_mGCC_ITxvsINx | $2.3 \pm 0.8$ | $1.7 \pm 0.6$ | <.01 |
|    log_mGCC_ITyvsINy | $2.5 \pm 0.8$ | $2.0 \pm 0.6$ | <.01 |
| Axial length (mm) | $25.93 \pm 1.71$ | $24.43 \pm 1.36$ | <.01 |

Fig. 6

Fig. 7

| Intercept and Retinal thickness-related predictors | B | SE | OR | 95% CI | P Value |
|---|---|---|---|---|---|
| **Model 1** | | | | | |
| Intercept | 14.4305 | 2.1692 | – | – | <.01 |
| TSNITlower | −0.0404 | 0.0153 | 0.96 | 0.93 −0.99 | .01 |
| cpRNFLqS | −0.0303 | 0.0127 | 0.97 | 0.95 −0.99 | .02 |
| cpRNFLqIt | −0.0304 | 0.0097 | 0.97 | 0.95 −0.99 | <.01 |
| cpRNFLqIn | −0.0271 | 0.0096 | 0.97 | 0.96 −0.99 | <.01 |
| mGCIPL_IT | −0.1424 | 0.0457 | 0.87 | 0.79 −0.95 | <.01 |
| log_mGCIPL_STvsIT | 1.1427 | 0.2408 | 3.14 | 1.96 −5.03 | <.01 |
| log_mGCIPL_ITvsIN | 0.6971 | 0.2968 | 2.01 | 1.12 −3.59 | .02 |
| mGCC_IN | 0.0602 | 0.0270 | 1.06 | 1.01 −1.12 | .03 |
| AUC-ROC (95%CI) | 0.971 (0.960−0.983) | | | | |
| **Model 2** | | | | | |
| Intercept | 12.6694 | 1.5639 | – | – | <.01 |
| TSNITlower | −0.0329 | 0.0150 | 0.97 | 0.94 −1.00 | .03 |
| cpRNFLqS | −0.0344 | 0.0126 | 0.97 | 0.94 −0.99 | .01 |
| cpRNFLqIt | −0.0318 | 0.0078 | 0.97 | 0.95 −0.98 | <.01 |
| cpRNFLqIn | −0.0398 | 0.0091 | 0.96 | 0.94 −0.98 | <.01 |
| log_mGCIPL_STxvsITx | 1.1646 | 0.2164 | 3.21 | 2.10 −4.90 | <.01 |
| log_mGCC_ITxvsINx | 0.7120 | 0.2425 | 2.04 | 1.27 −3.28 | <.01 |
| AUC-ROC (95%CI) | 0.969 (0.957−0.982) | | | | |
| **Model 3** | | | | | |
| Intercept | 12.5935 | 1.7128 | – | – | <.01 |
| TSNITlower | −0.0660 | 0.0108 | 0.94 | 0.92 −0.96 | <.01 |
| cpRNFLqIn | −0.0296 | 0.0087 | 0.97 | 0.95 −0.99 | <.01 |
| mGCC | −0.0289 | 0.0080 | 0.97 | 0.96 −0.99 | <.01 |
| log_mGCIPL_SNyvsINy | 0.9845 | 0.2073 | 2.68 | 1.78 −4.02 | <.01 |
| log_mGCIPL_STyvsITy | 0.5671 | 0.2719 | 1.76 | 1.04 −3.00 | .04 |
| log_mGCC_ITyvsINy | 0.6613 | 0.2450 | 1.94 | 1.20 −3.13 | .01 |
| AUC-ROC (95% CI) | 0.968 (0.955−0.981) | | | | |

Fig. 8

Model 1

Model 2

Model 3

AUC=0.971 (0.960 -0.983)

AUC=0.969 (0.957 -0.982)

AUC=0.968 (0.955 -0.981)

Fig. 9

| Calculation Model | Risk Score | Validation Data Set | | SD-OCT Report Diagnosed by Four Ophthalmologists | | |
|---|---|---|---|---|---|---|
| | | N | % | Total, N | Glaucoma, N | PPV, % |
| Model 1 | | | | | | |
| Low | 0 to 49 | 8380 | 86.2 | 157 | 19 | 12.1 |
| Middle | 50 to 89 | 750 | 7.7 | 183 | 110 | 60.1 |
| High | 90 to 100 | 590 | 6.1 | 383 | 309 | 80.7 |
| Model 2 | | | | | | |
| Low | 0 to 49 | 8339 | 85.8 | 155 | 18 | 11.6 |
| Middle | 50 to 89 | 776 | 8.0 | 167 | 86 | 51.5 |
| High | 90 to 100 | 605 | 6.2 | 401 | 334 | 83.3 |
| Model 3 | | | | | | |
| Low | 0 to 49 | 8280 | 85.2 | 186 | 22 | 11.8 |
| Middle | 50 to 89 | 842 | 8.7 | 168 | 81 | 48.2 |
| High | 90 to 100 | 598 | 6.2 | 369 | 335 | 90.8 |
| Total | | 9720 | 100 | 723 | 438 | 60.6 |

Fig. 10

| Diagnosis | Model 3 Risk Score | | |
|---|---|---|---|
| | High (90 to 100), *N* | Middle (50 to 89), *N* | Low (0 to 49), *N* |
| All, *N* | 67 | 17 | 45 |
| Glaucoma | 53 | 8 | 1 |
| Other eye disease | 0 | 3 | 4 |
| Undeterminable | 10 | 1 | 3 |
| Normal | 4 | 5 | 37 |

Fig. 11

| Model 3 Risk Score | Glaucoma | Normal | Total |
| --- | --- | --- | --- |
| 90 to 100 | 53 (85%) | 4 | 57 |
| 0 to 89 | 9 | 42 (91%) | 51 |
| Total | 62 | 46 | 108 |

Fig. 12

## INTERNATIONAL SEARCH REPORT

| International application No. |
|---|
| **PCT/JP2023/025587** |

**A. CLASSIFICATION OF SUBJECT MATTER**

*A61B 3/10*(2006.01)i
FI:   A61B3/10 100

According to International Patent Classification (IPC) or to both national classification and IPC

**B. FIELDS SEARCHED**

Minimum documentation searched (classification system followed by classification symbols)

A61B3/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2013-208395 A (CANON INC.) 10 October 2013 (2013-10-10)<br>entire text, all drawings | 1-18 |
| A | JP 2014-155875 A (TOPCON CORP.) 28 August 2014 (2014-08-28)<br>entire text, all drawings | 1-18 |
| A | JP 2021-184169 A (CANON INC.) 02 December 2021 (2021-12-02)<br>paragraph [0067] | 1-18 |
| A | WO 2019/073962 A1 (THE UNIV. OF TOKYO) 18 April 2019 (2019-04-18)<br>paragraphs [0072], [0075] | 1-18 |

☐ Further documents are listed in the continuation of Box C.    ☑ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **28 August 2023** | **05 September 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/025587**

| Patent document cited in search report | | | Publication date (day/month/year) | Patent family member(s) | Publication date (day/month/year) |
|---|---|---|---|---|---|
| JP | 2013-208395 | A | 10 October 2013 | US 2013/0258285 A1 entire text, all drawings | |
| JP | 2014-155875 | A | 28 August 2014 | (Family: none) | |
| JP | 2021-184169 | A | 02 December 2021 | (Family: none) | |
| WO | 2019/073962 | A1 | 18 April 2019 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5184542 A
- US 20180025112
- US 9295386 B
- US 11013400 B